# EUROPEAN PATENT SPECIFICATION

(11) **EP 1 854 875 B1**
(45) Date of publication and mention of the grant of the patent: **03.11.2010**
(21) Application number: 06290764.7
(22) Date of filing: 12.05.2006
(51) Int. Cl.: C12N 9/12, C12P 19/34, C12Q 1/68, C12N 5/10, C12N 1/21, C12N 1/15

(54) **Thermostable DNA polymerase of the archaeal ampullavirus ABV and its applications**
Thermostabile DNA Polymerase von archae Ampullavirus ABV und dessen Verwendungen
Polymérase d'ADN thermostable provenant de Ampullavirus ABV archeen et de ses applications

(43) Date of publication of application: 14.11.2007
(73) Proprietor: INSTITUT PASTEUR, 75015 Paris (FR)
(72) Inventor: Peng, Xu, 2820 Gentofte (DK); Haering, Monika, 84489 Burghausen (DE); Garrett, Roger, 3050 Humlebaek (DK); Prangishvili, David, 78000 Versailles (FR)
(74) Representative: Warcoin, Jacques

(56) References cited:
- US-A- 5 198 543
- PECENKOVÁ T ET AL: "Molecular phylogeny of phi29-like phages and their evolutionary relatedness to other protein-primed replicating phages and other phages hosted by Gram-positive bacteria" JOURNAL OF MOLECULAR EVOLUTION, SPRINGER VERLAG, NEW YORK, NY, US, vol. 48, 1999, pages 197-208, XP002230514 ISSN: 0022-2844
- NELSON J R: "PHI29 DNA POLYMERASE-BASED METHODS FOR GENOMICS APPLICATIONS" JOURNAL OF CLINICAL LIGAND ASSAY, CLINICAL LIGAND ASSAY SOCIETY, WAYNE, MI, US, vol. 25, no. 3, October 2002 (2002-10), pages 276-279, XP009041683 ISSN: 1081-1672
- HARING MONIKA ET AL: "Viral diversity in hot springs of Pozzuoli, Italy, and characterization of a unique archaeal virus, acidianus bottle-shaped virus, from a new family, the Ampullaviridae" JOURNAL OF VIROLOGY, vol. 79, no. 15, August 2005 (2005-08), pages 9904-9911, XP002401548 ISSN: 0022-538X
- WANG C X ET AL: "Pre-steady-state kinetics of RB69 DNA polymerase and its exo domain mutants: Effect of pH and thiophosphoryl linkages on 3'-5' exonuclease activity" BIOCHEMISTRY, vol. 43, no. 13, 6 April 2004 (2004-04-06), pages 3853-3861, XP002401549 ISSN: 0006-2960
- SATO ET AL: "Usefulness of repeated GenomiPhi, a phi29 DNA polymerase-based rolling circle amplification kit, for generation of large amounts of plasmid DNA" BIOMOLECULAR ENGINEERING, ELSEVIER, NEW YORK, NY, US, vol. 22, no. 4, October 2005 (2005-10), pages 129-132, XP005042325 ISSN: 1389-0344
- RODRIGUEZ I ET AL: "phi29 DNA Polymerase-Terminal Protein Interaction. Involvement of Residues Specifically Conserved Among Protein-primed DNA Polymerases" JOURNAL OF MOLECULAR BIOLOGY, LONDON, GB, vol. 337, no. 4, 2 April 2004 (2004-04-02), pages 829-841, XP004496013 ISSN: 0022-2836
- DEAN F B ET AL: "Rapid amplification of plasmid and phage DNA using Phi29 DNA polymerase and multiply-primed rolling circle amplification" GENOME RESEARCH, COLD SPRING HARBOR LABORATORY PRESS, WOODBURY, NY, US, vol. 11, no. 6, June 2001 (2001-06), pages 1095-1099, XP002223174 ISSN: 1088-9051

### SEQUENCE LISTING

<110> Institut Pasteur
<120> Thermostable DNA polymerase of the archaeal ampullavirus ABV and its applications
<130> D24232
<160> 11
<170> PatentIn version 3.3
<210> 1
   <211> 653
   <212> PRT
   <213> Ampullavirus ABV
<220>
   <223> Thermostable DNA polymerase
<400> 1
   Met Leu Gln Ile Leu Asn Asn Ala Asn Asp Lys Ile Gln Tyr Lys Leu
   1               5                   10                  15
   Asn Asp Phe Ser Asp Leu Thr Pro Ser Phe Lys Ile Lys Ser Gly Phe
               20                  25                  30
   Phe Thr Ile His Arg Ile Asn Tyr Glu Lys Leu Glu Glu Asn Glu Glu
           35                  40                  45
   Glu Lys Pro Arg Gln Ile Ser Ala Phe Asp Ile Glu Thr Asn Gly Arg
       50                  55                  60
   Glu Ile Phe Phe Gly Val Tyr Asp Gly Ser Glu Tyr Lys Tyr Val Ile
   65                  70                  75                  80
   Ile Arg Lys Pro Glu Asp Val Lys Asn Ala Leu Asp Leu Leu Thr Glu
                   85                  90                  95
   Glu Thr Tyr Phe Tyr Gly Asp Tyr Asp Leu Pro Val Ser Leu Ala Asn
               100                 105                 110
   Tyr Val Leu Leu Gly Lys His Ser Lys Phe Ala Lys Lys Ile Thr Gly
           115                 120                 125
   Glu Asn Tyr Phe Thr Thr Lys Asn Leu Arg Ile Lys Arg Tyr Lys Asn
       130                 135                 140
   Phe Tyr Lys Ile Leu Tyr Asn Gly Arg Ser Ile Asn Ser Ile Asn Leu
   145                 150                 155                 160
   Leu Gln Phe Tyr Ser Glu Ser Leu Tyr Glu Ala Tyr Ser Arg Tyr Tyr
                   165                 170                 175
   Thr Lys Leu Gln Glu Met Gly Phe Gln Val Phe Asp Glu Gln Thr Met
               180                 185                 190
   Lys Glu Trp Lys Glu Asp Lys Glu Lys Arg Ala Asn Phe Asp Lys Leu
           195                 200                 205
   Asp Phe Asn Asp Pro Lys Thr Ile Gln Glu Ile Ala Arg Tyr Asn Lys
       210                 215                 220
   Leu Asp Val Ile Ala Thr Tyr Gln Leu Ala Leu Leu Lys Asn Ser Leu
   225                 230                 235                 240
   Phe Gly Ile Lys Val Lys Ser Thr Leu Pro Arg Thr Ala Ile Thr Tyr
                   245                 250                 255
   Ile Ile Ser Gln Val Gln Ser Glu Phe Val Lys Gly Leu Arg Ser Tyr
               260                 265                 270
   Thr Glu Ile Glu Leu Thr Leu Lys Arg Leu Tyr Lys Gly Gly Leu Phe
           275                 280                 285
   Asp Ser Asn Glu Leu Gly Lys Phe Lys Lys Val Tyr Lys Tyr Asp Val
       290                 295                 300
   Asn Ser Met Tyr Pro Phe Met Met Ser Trp Leu Pro Glu Leu Glu Leu
   305                 310                 315                 320
   Val Glu Thr Gln Lys Gly Phe Glu Val Asn Glu Glu Pro Leu Leu Lys
                   325                 330                 335
   Gly Thr Gln Phe Asn Glu Asp Ala Lys Tyr Val Tyr Ile Tyr Lys Ile
               340                 345                 350
   Thr Leu Lys Gln Asp Arg Lys Tyr Val Ala Ser Lys Ala Asn Gly Met
           355                 360                 365
   Leu Leu Arg Met Met Tyr Asn Arg Gly Ser Phe Phe Asp Phe Glu Leu
       370                 375                 380
   Ala Asp Glu Lys His Glu Leu Ile Pro Asp Ile Lys Ile Val Gly Gln
   385                 390                 395                 400
   Tyr Tyr Thr Met Lys Phe Lys Ile Thr Lys His Arg Ile Phe Lys Asp
                   405                 410                 415
   Val Ile Tyr Asn Leu Tyr Asn Lys Arg Leu Gln Leu Lys Lys Glu Lys
               420                 425                 430
   Asn Pro Leu Glu Lys Val Tyr Lys Leu Ile Leu Asn Ser Ser Tyr Gly
           435                 440                 445
   Lys Phe Gly Glu Arg Ile Gly Phe Asn Ala Lys Phe Gln Asn Val Ile
       450                 455                 460
   Tyr Ala Ser Met Ile Thr Ala Leu Gly Arg Thr Phe Ile Gln Asn Val
   465                 470                 475                 480
   Asp Pro Asn Ala Ile Ser Tyr Leu Thr Asp Ser Val Ile Ser Lys Ala
                   485                 490                 495
   Pro Ile Lys Ser Glu Leu Val Gly Asp Gln Leu Gly Gln Leu Lys Gln
               500                 505                 510
   Glu Gly Val Gly Glu Ala Ile Val Ile Gly Asn Gly Gln Tyr Ile Leu
           515                 520                 525
   Ser Asp Pro Gln Glu Lys Met Ile Lys Leu Arg Gly Phe Asn Val Asp
       530                 535                 540
   Glu Ser Ile Ala Glu Lys Ile Ile Glu Tyr Val Gly Asn Tyr Leu Ala
   545                 550                 555                 560
   Lys Gly Lys Ile Val Arg Val Gln Ile Pro Thr Lys Ile Met Ile Arg
                   565                 570                 575
   Asn Leu Gln Gln Tyr Lys Ile Leu Ala Glu Lys Asp Ser Asn Val Leu
               580                 585                 590
   Met Gly Met Leu Ser Asn Gln Ile Lys Ile Phe Thr Pro Leu Asn Thr
           595                 600                 605
   Lys Gln Arg Tyr Thr Tyr Asn Val His Trp Phe Gly Ser Met Phe Arg
       610                 615                 620
   Asp Glu Gln Glu His Lys Glu Tyr Arg Lys Thr Trp Glu Lys Tyr Ile
   625                 630                 635                 640
   Glu Thr Ile Glu Pro Ile Asp Leu Asn Ser Ile Leu Ser
                   645                 650
<210> 2
   <211> 1962
   <212> DNA
   <213> Ampullavirus ABV
<220>
   <223> cDNA encoding the thermostable DNA polymerase
<400> 2
   atgctacaaa tcctcaataa cgcaaacgat aagatccaat acaagttaaa cgatttttca     60
   gacttaaccc cttcattcaa aatcaaaagc ggattcttta ctatacacag aatcaattat    120
   gaaaaactag aggaaaatga agaggaaaaa ccaagacaaa ttagcgcatt cgacatagag    180
   acaaacggaa gagaaatctt tttcggagtt tatgatggtt ctgaatacaa atacgtaata    240
   attagaaaac cagaagatgt taaaaatgcc cttgatcttt taacagaaga aacatacttc    300
   tatggagatt atgatttacc agtaagttta gctaattatg ttcttctagg caaacactca    360
   aaattcgcaa agaaaatcac tggagaaaac tacttcacaa ctaaaaacct tagaataaaa    420
   agatacaaga acttctacaa aattctctat aatggaagat caataaactc aataaaccta    480
   ctccaattct acagtgagag tttgtatgaa gcctacagta ggtattatac caaactacaa    540
   gaaatgggat ttcaagtctt tgatgaacaa acaatgaaag agtggaagga agacaaagaa    600
   aaaagggcta attttgacaa attagatttc aatgatccca agacaataca agaaatagct    660
   aggtataaca aacttgatgt aattgccact taccaattag cgcttctaaa gaacagtctc    720
   tttggaataa aagtaaagtc cacgcttcca agaaccgcta taacttacat tatttcacaa    780
   gttcagtcag agtttgtcaa aggattaaga tcatacactg agatagagct aacattaaag    840
   agactttata agggcgggtt attcgactca aacgaattag gtaaattcaa aaaagtttac    900
   aagtatgacg tcaattcaat gtatccattc atgatgagtt ggttgccaga attagaatta    960
   gttgaaacac aaaaaggttt tgaagtaaac gaagaaccac tacttaaggg cacacaattc   1020
   aatgaagatg caaagtatgt ttacatttac aaaatcacat tgaaacaaga cagaaagtat   1080
   gtcgcttcaa aagctaatgg gatgcttctc agaatgatgt acaacagagg ctcattcttt   1140
   gatttcgaac ttgcagacga aaagcatgaa ctaataccag acattaaaat agtgggacaa   1200
   tactacacaa tgaaattcaa aataacaaaa catagaatct tcaaagatgt tatctataac   1260
   ctatacaaca agagattaca actaaagaaa gagaaaaacc cactagagaa agtttacaag   1320
   ttaattctaa acagttcata cgggaaattc ggcgaaagaa taggattcaa tgcaaagttc   1380
   cagaatgtaa tttacgcttc aatgattacc gctttaggaa gaacctttat ccaaaacgta   1440
   gaccctaacg ctataagtta tttaactgac agtgtaataa gcaaagctcc cataaagtct   1500
   gaactagtag gagaccaatt aggacaatta aaacaagaag gtgtaggaga agcaattgta   1560
   attggtaatg gtcagtacat tttgagcgac ccacaagaaa aaatgataaa gctaagagga   1620
   ttcaatgttg atgagtcaat tgccgaaaaa attattgaat acgtaggaaa ctatttagcc   1680
   aagggtaaaa ttgttagagt acaaattcca acaaagataa tgataagaaa cttacaacaa   1740
   tacaagattt tagccgaaaa agatagcaat gtgttaatgg gaatgctttc aaatcagatc   1800
   aaaatcttca caccacttaa tactaaacaa aggtatactt acaacgtaca ctggttcgga   1860
   agcatgttca gagacgaaca agaacataaa gaatacagaa agacatggga aaaatacata   1920
   gaaacaattg aaccaataga cttaaatagt attctctcat aa                      1962
<210> 3
   <211> 163
   <212> PRT
   <213> Ampullavirus ABV
<220>
   <223> Terminal protein of the ampullavirus ABV encoded by the ORF163 of the ABV genome
<400> 3
   Val Met Ile Lys Asp Leu Glu Leu Thr Leu Lys Asn Leu Val Ser Ser
   1               5                   10                  15
   Asn Leu Asp Ser Asn Cys Val His Val Thr Asn Met Ile Ser Tyr Gly
               20                  25                  30
   Asp Pro Pro Thr Gln Glu Met Leu Val Arg Gly Lys Leu Tyr His Leu
           35                  40                  45
   Ala Ile Glu Asn Leu Ile Gln Gln Val Phe Lys Asp Ala Ile Ile Glu
       50                  55                  60
   Gln Ser His Arg Asp Asn Ile Gly Asn Lys Glu Ile Cys Tyr Thr Pro
   65                  70                  75                  80
   Asp Val Tyr Val Pro Ser Lys Lys Leu Leu Ile Glu Ile Lys Ser Ser
                   85                  90                  95
   Asp Arg Ser Leu Ala Tyr Ala Thr Lys Gln Thr Ser Ile Tyr Arg Tyr
               100                 105                 110
   Leu Leu Glu Arg Lys Gly Val Ser Val Asp Gln Cys Ile Met Ile Thr
           115                 120                 125
   Gly Asp Leu Lys Val Phe Pro Leu Gln Cys Asp Ser Asn Tyr Gly Gln
       130                 135                 140
   Gln Leu Leu Asn Ser Tyr Leu Asn Ser Arg Leu Asp Gln Asn Leu Phe
   145                 150                 155                 160
   Lys Pro Lys
<210> 4
   <211> 492
   <212> DNA
   <213> Ampullavirus ABV
<220>
   <223> cDNA encoding the terminal protein of the ampullavirus ABV
<400> 4
   gtgatgataa aagatctaga attaaccctc aaaaatcttg tttcaagcaa tctcgatagc    60
   aattgcgtac acgttaccaa catgatctct tacggagatc ctccaacaca agaaatgtta   120
   gttagaggaa aactttacca tttagcaata gagaacctaa ttcaacaagt tttcaaagac   180
   gctattatag aacagtccca tagagacaac ataggtaata aagagatttg ttatacacca   240
   gacgtttatg taccttccaa gaaactctta atagagatta aatcctctga cagaagcctg   300
   gcttatgcca caaaacaaac ttccatttac cgttatcttc tagaaagaaa aggcgtatca   360
   gttgaccaat gtataatgat aaccggcgac ttgaaagtat tcccccttca atgcgatagt   420
   aattacggac aacaactctt aaactcatac ctaaactccc gattagacca aaatcttttt   480
   aaacctaaat ag                                                       492
<210> 5
   <211> 600
   <212> DNA
   <213> Ampullavirus ABV
<220>
   <223> Sequence of the left end of the Ampullavirus ABV genomic termini including the Inverted Terminal Repeat
<400> 5
   ggggagtcag tggagcatta gatttctatt aattagatta tccctaacta attattattt     60
   attctctctt attattttta tgtctcgccc cttttctttc ggggcatttc ttttttctca     120
   agtaatgtat gtgcaaatca agtataaaaa tctttctgaa ctgttgttgt aacctgcttg    180
   agtatgtgag tgagagttga tagcgttatt accgtctgtt tgtgttgttc taagggtagt    240
   ggaaaaaatg taaggtgaag cggtgaaggg tttctgtgta actgttgtca tgattctctg    300
   tgaatgtatt gtgtgaaact ggtatttaag ctttttggta tttgtgtcat gagttgttgt     360
   catgtgtctc tggttctaat gtgtgtggta aaggtttaaa aaactttcgg tttgtgagtg    420
   ggtattattg ttgagtgttt gtgtttgagc taggttttgt gtttgtgctt gttgtgttat    480
   tggattgttg gattgttgta tcattgtttg tctgttttgt tcttatctta ctgggattat    540
   ttaaagcttt ctgtgacctg gaaggtgaat aggatttaat agagagaaaa tttggaatgt    600
<210> 6
   <211> 600
   <212> DNA
   <213> Ampullavirus ABV
<220>
   <223> Sequence of the right end of the Ampullavirus ABV genomic termini including the Inverted Terminal Repeat
<400> 6
   acgttaacgg cgggagaaaa attaaatcct attcaccttc caggtcacag aaagctttaa     60
   ataatcccag taagataaga acaaaacaga caaacaatga tacaacaatc caacaatcca    120
   ataacacaac aagcacaaac acaaaaccta gctcaaacac aaacactcaa caataatacc    180
   cactcacaaa ccgaaagttt tttaaacctt taccacacac attagaacca gagacacatg    240
   acaacaactc atgacacaaa taccaaaaag cttaaatacc agtttcacac aatacattca    300
   cagagaatca tgacaacagt tacacagaaa cccttcaccg cttcacctta cattttttcc     360
   actaccctta gaacaacaca aacagacggt aataacgcta tcaactctca ctcacatact    420
   caagcaggtt acaacaacag ttcagaaaga tttttatact tgatttgcac atacattact    480
   tgagaaaaaa gaaatgcccc gaaagaaaag gggcgagaca taaaaataat aagagagaat    540
   aaataataat tagttaggga taatctaatt aatagaaatc taatgctcca ctgactcccc    600
<210> 7
   <211> 575
   <212> PRT
   <213> Bacillus phage phi29
<220>
   <223> DNA polymerase type-B family (from GenBank Accession number 1XI1B)
<400> 7
   Met Lys His Met Pro Arg Lys Met Tyr Ser Cys Ala Phe Glu Thr Thr
   1               5                   10                  15
   Thr Lys Val Glu Asp Cys Arg Val Trp Ala Tyr Gly Tyr Met Asn Ile
               20                  25                  30
   Glu Asp His Ser Glu Tyr Lys Ile Gly Asn Ser Leu Asp Glu Phe Met
           35                  40                  45
   Ala Trp Val Leu Lys Val Gln Ala Asp Leu Tyr Phe His Asn Leu Lys
       50                  55                  60
   Phe Ala Gly Ala Phe Ile Ile Asn Trp Leu Glu Arg Asn Gly Phe Lys
   65                  70                  75                  80
   Trp Ser Ala Asp Gly Leu Pro Asn Thr Tyr Asn Thr Ile Ile Ser Arg
                   85                  90                  95
   Met Gly Gln Trp Tyr Met Ile Asp Ile Cys Leu Gly Tyr Lys Gly Lys
               100                 105                 110
   Arg Lys Ile His Thr Val Ile Tyr Asp Ser Leu Lys Lys Leu Pro Phe
           115                 120                 125
   Pro Val Lys Lys Ile Ala Lys Asp Phe Lys Leu Thr Val Leu Lys Gly
       130                 135                 140
   Asp Ile Asp Tyr His Lys Glu Arg Pro Val Gly Tyr Lys Ile Thr Pro
   145                 150                 155                 160
   Glu Glu Tyr Ala Tyr Ile Lys Asn Asp Ile Gln Ile Ile Ala Glu Ala
                   165                 170                 175
   Leu Leu Ile Gln Phe Lys Gln Gly Leu Asp Arg Met Thr Ala Gly Ser
               180                 185                 190
   Asp Ser Leu Lys Gly Phe Lys Asp Ile Ile Thr Thr Lys Lys Phe Lys
           195                 200                 205
   Lys Val Phe Pro Thr Leu Ser Leu Gly Leu Asp Lys Glu Val Arg Tyr
       210                 215                 220
   Ala Tyr Arg Gly Gly Phe Thr Trp Leu Asn Asp Arg Phe Lys Glu Lys
   225                 230                 235                 240
   Glu Ile Gly Glu Gly Met Val Phe Asp Val Asn Ser Leu Tyr Pro Ala
                   245                 250                 255
   Gln Met Tyr Ser Arg Leu Leu Pro Tyr Gly Glu Pro Ile Val Phe Glu
               260                 265                 270
   Gly Lys Tyr Val Trp Asp Glu Asp Tyr Pro Leu His Ile Gln His Ile
           275                 280                 285
   Arg Cys Glu Phe Glu Leu Lys Glu Gly Tyr Ile Pro Thr Ile Gln Ile
       290                 295                 300
   Lys Arg Ser Arg Phe Tyr Lys Gly Asn Glu Tyr Leu Lys Ser Ser Gly
   305                 310                 315                 320
   Gly Glu Ile Ala Asp Leu Trp Leu Ser Asn Val Asp Leu Glu Leu Met
                   325                 330                 335
   Lys Glu His Tyr Asp Leu Tyr Asn Val Glu Tyr Ile Ser Gly Leu Lys
               340                 345                 350
   Phe Lys Ala Thr Thr Gly Leu Phe Lys Asp Phe Ile Asp Lys Trp Thr
           355                 360                 365
   Tyr Ile Lys Thr Thr Ser Glu Gly Ala Ile Lys Gln Leu Ala Lys Leu
       370                 375                 380
   Met Leu Asn Ser Leu Tyr Gly Lys Phe Ala Ser Asn Pro Asp Val Thr
   385                 390                 395                 400
   Gly Lys Val Pro Tyr Leu Lys Glu Asn Gly Ala Leu Gly Phe Arg Leu
                   405                 410                 415
   Gly Glu Glu Glu Thr Lys Asp Pro Val Tyr Thr Pro Met Gly Val Phe
               420                 425                 430
   Ile Thr Ala Trp Ala Arg Tyr Thr Thr Ile Thr Ala Ala Gln Ala Cys
           435                 440                 445
   Tyr Asp Arg Ile Ile Tyr Cys Asp Thr Asp Ser Ile His Leu Thr Gly
       450                 455                 460
   Thr Glu Ile Pro Asp Val Ile Lys Asp Ile Val Asp Pro Lys Lys Leu
   465                 470                 475                 480
   Gly Tyr Trp Ala His Glu Ser Thr Phe Lys Arg Ala Lys Tyr Leu Arg
                   485                 490                 495
   Gln Lys Thr Tyr Ile Gln Asp Ile Tyr Met Lys Glu Val Asp Gly Lys
               500                 505                 510
   Leu Val Glu Gly Ser Pro Asp Asp Tyr Thr Asp Ile Lys Phe Ser Val
           515                 520                 525
   Lys Cys Ala Gly Met Thr Asp Lys Ile Lys Lys Glu Val Thr Phe Glu
       530                 535                 540
   Asn Phe Lys Val Gly Phe Ser Arg Lys Met Lys Pro Lys Pro Val Gln
   545                 550                 555                 560
   Val Pro Gly Gly Val Val Leu Val Asp Asp Thr Phe Thr Ile Lys
                   565                 570                 575
<210> 8
   <211> 18
   <212> DNA
   <213> artificial sequence
<220>
   <223> Primer used as primer-template DNA molecule in the polymerization of the hybrid molecule polF1/polF1c+24
<400> 8
   cctccctatt tgataggc 18
<210> 9
   <211> 42
   <212> DNA
   <213> artificial sequence
<220>
   <223> Primer used as primer-template DNA molecule in the polymerization of the hybrid molecule polF1/polF1c+24
<400> 9
   aggtaagcat gcatcagtta atacgcctat caaataggga gg 42
<210> 10
   <211> 25
   <212> DNA
   <213> artificial sequence
<220>
   <223> Primer used as primer-template DNA molecule for the amplification of the DNA polymerase of Ampullavirus ABV
<400> 10
   tatttttaca tatgctacaa atcct 25
<210> 11
   <211> 32
   <212> DNA
   <213> artificial sequence
<220>
   <223> Primer used as primer-template DNA molecule for the amplification of the DNA polymerase of Ampullavirus ABV
<400> 11
   tataactcga gtgagagaat actatttaag tc 32

## Description

The present invention is directed to the thermostable DNA polymerase protein of the archaeal ampullavirus ABV (Acidianus Bottle-shaped virus) and the nucleic acid encoding said DNA polymerase. The invention also relates to method of synthesizing, amplifying or sequencing nucleic acid implementing said DNA polymerase protein and kit or apparatus comprising said DNA polymerase protein.

The double-stranded (ds) DNA viruses of hyperthermophilic *Crenarchaeota* exhibit remarkably diverse morphotypes and genome structures and, on the basis of these properties several have already been assigned to six new viral families: spindle-shaped *Fuselloviridae,* filamentous *Lipothrixviridae,* rod-shaped *Rudiviridae,* droplet-shaped *Guttaviridae,* spherical *Globuloviridae* and two-tailed *Bicaudaviridae* (reviewed in Prangishvili et al., 2001; Prangishvili and Garrett, 2004, 2005). A novel virus was recently discovered which exhibited a unique bottle-shaped morphology and it was tentatively assigned to a new family, the Ampullaviridae (Häring et al., 2005a).

A variety of nucleic acid amplification techniques, developed as tools for nucleic acid analysis and manipulation, have been successfully applied for clinical diagnosis of genetic and infectious diseases. Amplification techniques can be grouped into those requiring temperature cycling (PCR and ligase chain reaction) and isothermal systems (amplification systems (3SR and NASBA), strand-displacement amplification, and Qβ replication systems). Two aspects are frequent caveats in these procedures: fidelity of synthesis and length of the amplified product.

Development of an amplification system relying on the mechanism of phage phi29 (φ29) DNA replication has been the object of publications and patent documents (Dean et al., Genome Res. 2001 Jun;11 (6):1095-9; Mendez et al., EMBO J. 1997 1;16(9):2519-27; Hutchison et al., Proc Natl Acad Sci U S A. 2005;102(48):17332-6; Mamone, Innovations Forum: GenomiPhi DNA amplification, Life Sciences News 14, 2003 Amersham Biosciences; Blanco et al., 1994; EP 0 862 656 or U.S. 5,001,050).

The phi29 DNA polymerase is a highly processive polymerase featuring strong strand displacement activity which allows for highly efficient isothermal DNA amplification (Blanco et al., Proc. Natl. Acad. Sci. USA, 81, 5325-5329, 1984 and J. Biol.Chem., 264, 8935-8940, 1989). The phi29 DNA Polymerase also possesses a 3'=>5' exonuclease (proofreading) activity acting preferentially on single-stranded DNA (Garmendia J. Biol.Chem., 267, 2594-2599, 1992).

Among its features, we can cited its highest processivity and strand displacement activity among known DNA polymerases - more than 70 kb long DNA stretches can be synthesized (Blanco et al., 1989), its highly accurate DNA synthesis (Esteban et al., J. Biol. Chem., 268, 4, 2719-2726, 1993), its high yields of amplified DNA even from minute amounts of template and the amplification products can be directly used in downstream applications (PCR, restriction digestion, SNP genotyping, etc.). Numerous specific applications were developed implementing this particular DNA polymerase such as Rolling Circle Amplification (RCA) (Lizardi et al., Nat. Genet., 19, 225-232, 1998; Dean et al., Genome Res., 11, 1095-1099, 2001; Baner et al., Nucleic Acids Res., 26, 5073-5078, 1998). Multiple Displacement amplification (MDA) (Dean et al., Proc. Natl. Acad. Sci. USA, 99, 5261-5266, 2002), unbiased amplification of whole genome or DNA template preparation for sequencing. This system would be adequate for faithful amplification of DNA molecules longer than 70 kb (Blanco et al., 1989), largely over the size limit obtained with the amplification systems available to date. This procedure of isothermal TP-primed amplification ("TP" for terminal protein) would exploit the particular properties of phi29 DNA polymerase: (1) ability to use a protein as primer, (ii) intrinsic high processivity (>70 kb), and (iii) strand displacement coupled to DNA synthesis. The specific activity for this phi29 DNA polymerase is given for a temperature of 30°C and it is precised that this phi29 DNA polymerase is inactivated at 65°C.

Currently there is a need for a new DNA polymerase belonging to the protein-primed DNA polymerase family such as phi29 DNA polymerase which can work at temperature significantly superior to 30°C and which is not completely inactivated at 60°C.

We can cite the document US 5,198,543 (Blanco et al., 1993) which discloses a Phi 29 DNA polymerase which can be used in a method for determining the nucleotide base sequence of a DNA molecule.

We can also cite the document Pecenkova et al. (J. Mol. Evol., 48:197-208, 1999) which studies and analyses the nucleotide and amino acid sequences of selecte"d DNA regions and proteins of the Phi 29 - like phage genus in order to assess phylogeny of the phage group, particularly of terminal proteins and DNA polymerases coding regions.

This is the object of the present invention.

After sequencing and annoted the complete genomic sequence of the virus ABV (Acidianus Bottle-shaped virus) infecting hyperthermophilic archaea of the genera Acidianus, the inventors have demonstrated a nucleic sequence encoding a DNA-dependent DNA polymerase. Surprisingly, the nalysis of the protein sequence indicated that it belongs to the protein-primed DNA polymerase family. The gene for DNA polymerase was heterologously expressed in E. coli and DNA polymerization activity of the recombinant protein has been confirmed. This novel enzyme, similar to known viral DNA polymerases, is highy processive and self sufficient, not requiring auxiliary proteins. Due to these features the enzyme can have significant advantages as a tool for DNA amplification by PCR. Being protein-primed thermostable viral enzyme it can be much more efficient in exponential amplification of single- or double-stranded linear DNA (i.e. by the GenomiPhi procedure developed by Amersham) than bacteriophage Phi29 DNA polymerase, a mesophilic protein-primed enzyme, currently utilized in this procedure. GenomiPhi Amplification Kit of Amersham enables to perform unlimited DNA tests from a small number of cells or limited amount of precious sample and is an easy genomic DNA amplification method that representatively amplifies the whole genome.

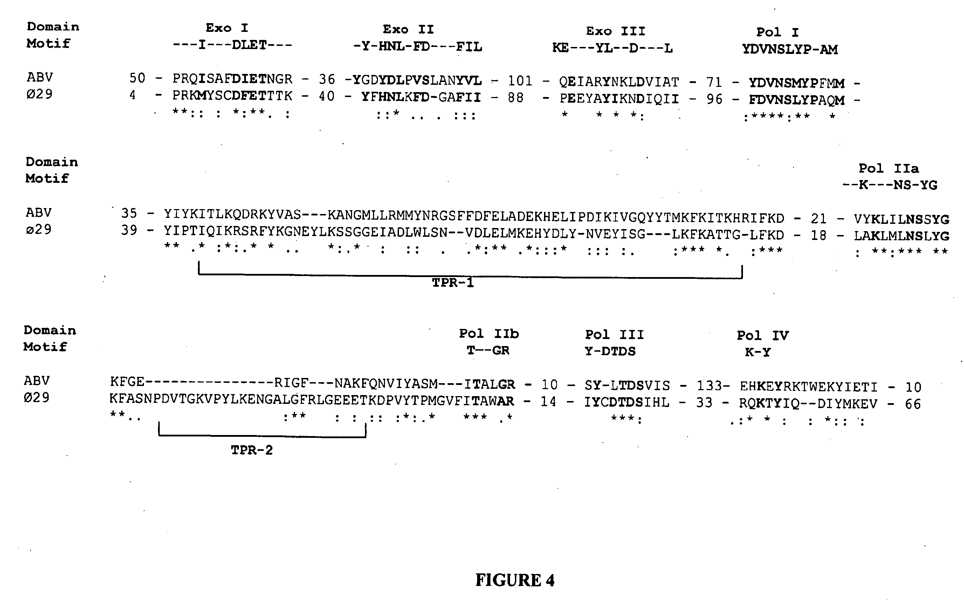

So, in a first aspect, the present invention is directed to an isolated DNA polymerase selected from the group of polypeptides consisting of:
a) the polypeptide having the amino acid sequence of SEQ ID NO: 1;
b) a fragment of a) having a DNA polymerase activity;
c) a polypeptide comprising at least the SEQ ID NO: 1 fragments allowing the DNA polymerase activity of said DNA polymerase of a);
d) a polypeptide having the amino acid sequence of SEQ ID NO: 1 wherein the exonuclease sites Exo I, Exo II and/or Exo III as identified in Figure 4 have been mutated or deleted to result in a DNA polymerase polypeptide having a deficient or no detectable exonuclease activity compared to the polypeptide having the amino acid sequence of SEQ ID NO: 1;
e) a polypeptide having sequence which is at least 80 % identity after optimum alignment with the sequence SEQ ID NO: 1, or as defined in b) to d), said polypeptide having a DNA polymerase activity. It is disclosed that said polypeptide has a DNA polymerase activity at a temperature of 50°C or superior to 50°C.

In a preferred embodiment, the DNA polymerase according to the invention is isolated from the archaeal Ampullavirus ABV. It is disclosed that said DNA polymerase is isolated from the ABV gene encoding the DBA polymerase.

In a more preferred embodiment, the DNA polymerase of the present invention comprises at least the Pol I, Pol IIa, Pol IIb, Pol III and Pol IV fragments of SEQ ID NO: 1 as identified in Figure 4.

Referring to the Figure 4, the polypeptide of the present invention having its DNA polymerase preserved but a deficient or significantly less exonuclease activity than the polypeptide having the sequence SEQ ID NO: 1 can be selected by taking into account the amino acid sequence homology with other polymerases and those mutations known to reduce exonuclease activity of DNA polymerase (Derbyshire et al., Science. 1988 Apr 8; 240(4849):199-201). Generally, the amino acid at these portions shown as Exo I, Exo II and/or Exo III in figure 4 can be either deleted or replaced with different amino acids. Large deletions or multiple replacement of amino acids at these Exo I, Exo II and/or Exo III positions can be also carried out. After mutagenesis the polypeptide having the sequence SEQ ID NO: 1, the level of exonuclease activity is measured and the amount of DNA polymerase activity determined to ensure it is sufficient for use in the present invention.

The term "5' exonuclease activity" refers to the presence of an activity in a protein which is capable of removing nucleotides from the 5' end of an oligonucleotide. 5' exonuclease activity may be measured using any of the assays provided herein.

The DNA polymerases of this invention include polypeptides which have been genetically modified to reduce the exonuclease activity of that polymerase, as well as those which are substantially identical (identity to at least 80 %) naturally-occurring ABV DNA polymerase or a modified polymerase thereof, or to the equivalent enzymes enumerated above. Each of these enzymes can be modified to have properties similar to those of the ABV DNA polymerase. It is possible to isolate the enzyme from ABV virus infected cells directly, but preferably the enzyme is isolated from cells which overproduce it (recombinant expression).

The term "exonuclease activity" refers to the presence of an activity in a protein which is capable of removing nucleotides from the 3' end or from the 5' end of an oligonucleotide. Such exonuclease activity may be measured using any of the exonuclease activity assays well known by the skilled person.

The term "DNA polymerase activity" refers to the ability of an enzymatic polypeptide to synthesize new DNA strands by the incorporation of deoxynucleoside triphosphates. The example 4 below provides an example of assay for the measurement of DNA polymerase activity. Such DNA polymerase activity may be measured using any of the DNA polymerase activity assays well known by the skilled person. A protein which can direct the synthesis of new DNA strands (DNA synthesis) by the incorporation of deoxynucleoside triphosphates in a template-dependent manner is said to be "capable of DNA polymerase activity".

In the present description, the terms polypeptides, polypeptide sequences, peptides and proteins are interchangeable.

The terms "identical" or percent "identity", in the context of two or more polypeptide sequences, refer to two or more sequences or subsequences that are the same or have a specified percentage of amino acid residues that are the same (i.e., about 80 % identity, preferably 85 %, 90 %, 95 %, 98 %, 99 %, or higher identity over a specified region when compared and aligned for maximum correspondence over a comparison window or designated region) as measured using a BLAST or BLAST 2.0 sequence comparison algorithms with default parameters, or by manual alignment and visual inspection (see, e.g., NCBI web site). The definition also includes sequences that have deletions and/or additions, as well as those that have substitutions. As described below, the preferred algorithms can account for gaps and the like. Preferably, identity exists over a region that is at least about 25 amino acids in length, or more preferably over a region that is 25-75 amino acids in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

Methods of alignment of sequences for comparison are well-known in the art. A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990).

For example, it is possible to use the BLAST program, "BLAST 2 sequences" (Tatusova et al., "Blast 2 sequences - a new tool for comparing protein and nucleotide sequences", FEMS Microbiol Lett. 174:247-250) available on the site http://www.ncbi.nlm.nih.gov/ gorf/b12.html, the parameters used being those given by default (in particular for the parameters "open gap penalty": 5, and "extension gap penalty": 2; the matrix chosen being, for example, the matrix "BLOSUM 62" proposed by the program), the percentage of identity between the two sequences to be compared being calculated directly by the program.

By amino acid sequence having at least 80 %, preferably 85 %, 90 %, 95 %, 98 %, 99 %, or higher identity with a reference amino acid sequence, those having, with respect to the reference sequence, certain modifications, in particular a deletion, addition or substitution of at least one amino acid, a truncation or an elongation are preferred. In the case of a substitution of one or more consecutive or nonconsecutive amino acid(s), the substitutions are preferred in which the substituted amino acids are replaced by "equivalent" amino acids. The expression "equivalent amino acids" is aimed here at indicating any amino acid capable of being substituted with one of the amino acids of the base structure without, however, essentially modifying the DNA polymerase activity of the reference polypeptide and such as will be defined later, especially in the example 4, last paragraph.

These equivalent amino acids can be determined either by relying on their structural homology with the amino acids which they replace, or on results of comparative trials of DNA polymerase activity between the different polypeptides capable of being carried out. By way of example, mention is made of the possibilities of substitution capable of being carried out without resulting in a profound modification of the DNA polymerase activity of the corresponding modified polypeptide. It is thus possible to replace leucine by valine or isoleucine, aspartic acid by glutamic acid, glutamine by asparagine, arginine by lysine, etc., the reverse substitutions being naturally envisageable under the same conditions.

So, in a second aspect, the present invention provides a nucleic acid encoding a DNA polymerase polypeptide according to the invention.

It is disclosed the nucleic acid having the sequence SEQ ID NO: 2 or having a sequence which is at least 80 % identity after optimum alignment with the sequence SEQ ID NO: 2, the polypeptide encoded by said nucleic acid having a DNA polymerase activity, preferably at a temperature of 50°C or superior to 50°C.

In the present description, the terms nucleic acid, polynucleotide, oligonucleotide, or acid nucleic or nucleotide sequence are interchangeable.

In another aspect, the invention encompasses a vector comprising the nucleic acid of the invention.

In a preferred embodiment, the vector according to the invention is **characterized in that** said nucleic acid is operably linked to a promoter.

The present disclosure relates to cloning and/or expression vectors which contain a nucleotide sequence according to the invention.

It is disclosed that the vectors according to the invention contain elements which allow the expression and/or the secretion of the nucleotide sequences in a determined host cell. The vector must therefore contain a promoter, signals of initiation and termination of translation, as well as appropriate regions of regulation of transcription. It must be able to be maintained in a stable manner in the host cell and can optionally have particular signals which specify the secretion of the translated protein. These different elements are chosen and optimized by the person skilled in the art as a function of the host cell used. To this effect, the nucleotide sequences according to the invention can be inserted into autonomous replication vectors in the chosen host, or be integrative vectors of the chosen host.

Such vectors are prepared by methods currently used by the person skilled in the art, and the resulting clones can be introduced into an appropriate host by standard methods, such as lipofection electroporation, thermal shock, or chemical methods.

The vectors can be, for example, vectors of plasmidic or viral origin. They are useful for transforming host cells in order to clone or to express the nucleotide sequences according to the invention.

In a preferred embodiment, the vector of the present invention is the plasmidic vector contained in the bacteria which has been deposited according to the Budapest Treaty at the C.N.C.M. (Collection Nationale de Cultures de Microorganismes, Institut Pasteur, Paris, France) the 28 April 2006 under the number I-3601.

This cloned plasmidic vector is the vector pET30a wherein the nucleic sequence of the DNA polymerase of the invention has been inserted between the NdeI and XbaI sites of the pET30a plasmid.

The term "expression vector" refers to a recombinant DNA molecule containing the desired coding nucleic acid sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

In another aspect, the present invention relates to a host cell comprising the vector according to the invention, particularly the recombinant bacteria which has been deposited according to the Budapest Treaty at the C.N.C.M. (Collection Nationale de Cultures de Microorganismes, Institut Pasteur, Paris, France) the 28 April 2006 under the number I-3601.

The DNA polymerase polypeptide of the present invention may be expressed in either prokaryotic or eukaryotic host cells. Nucleic acid encoding the DNA polymerase polypeptide of the present invention may be introduced into bacterial host cells by a number of means including transformation of bacterial cells made competent for transformation by treatment with calcium chloride or by electroporation. If the DNA polymerase polypeptide of the present invention are to be expressed in eukaryotic host cells, nucleic acid encoding the DNA polymerase polypeptide of the present invention may be introduced into eukaryotic host cells by a number of means including calcium phosphate co-precipitation, spheroplast fusion, electroporation and the like. When the eukaryotic host cell is a yeast cell, transformation may be affected by treatment of the host cells with lithium acetate or by electroporation or any other method known in the art. It is contemplated that any host cell will be useful in producing the peptides or proteins or fragments thereof of the invention.

The cells transformed according to the invention can be used in processes for preparation of recombinant polypeptides according to the invention. The processes for preparation of a polypeptide according to the invention in recombinant form, **characterized in that** they employ a vector and/or a cell transformed by a vector according to the invention, are themselves comprised in the present invention.

Preferably, a cell transformed by a vector according to the invention is cultured under conditions which allow the expression of said polypeptide and said recombinant peptide is recovered.

In another aspect, the present invention relates to a method of producing a DNA polymerase, said method comprising:
(a) culturing the host cell according to the invention in conditions suitable for the expression of said nucleic acid; and
(b) isolating said DNA polymerase from said host cell.

Said host cell can be a prokaryotic or an eukaryotic cell.

As has been said, the host cell can be chosen from prokaryotic or eukaryotic systems. In particular, it is possible to use nucleotide sequences facilitating secretion in such a prokaryotic or eukaryotic system. A vector according to the invention carrying such a sequence can therefore advantageously be used for the production of recombinant proteins, intended to be secreted. In effect, the purification of these recombinant proteins of interest will be facilitated by the fact that they are present in the supernatant of the cell culture rather than in the interior of the host cells.

In another aspect, the present invention encompasses a method of synthesizing a double-stranded DNA molecule comprising:
(a) hybridizing a primer to a first DNA molecule; and
(b) incubating said DNA molecule of step (a) in the presence of one or more deoxyribonucleoside triphosphates or analogs thereof and the polypeptide according to the invention, under conditions sufficient to synthesize a second DNA molecule complementary to all or a portion of said first DNA molecule.

In another aspect, the present invention encompasses a method of synthesizing a single-stranded DNA molecule comprising:
(a) the synthesis of a double-stranded DNA molecule by a method according to the invention; and
(b) denaturing the double-stranded DNA molecule obtained in step (a); and
(c) recovering the single-stranded DNA molecule obtained in step (b).

In another aspect, the present invention encompasses a method for production of DNA molecules of greater than 10 kilobases in length comprising the method according to the invention, wherein the first DNA molecule:
which serve as a template in step (a) is greater than 10 kilobases.

In the method according to the invention, said deoxyribonucleoside triphosphates are selected from the group consisting of dATP, dCTP, dGTP and dTTP.

In another aspect, the present invention encompasses a method for amplifying a double stranded DNA molecule, comprising:
(a) providing a first and second primer, wherein said first primer is complementary to a sequence at or near the 3'-termini of the first strand of said DNA molecule and said second primer is complementary to a sequence at or near the 3'-termini of the second strand of said DNA molecule;
(b) hybridizing said first primer to said first strand and said second primer to said second strand in the presence of the polypeptide according to the invention, under conditions such that a nucleic acid complementary to said first strand and a nucleic acid complementary to said second strand are synthesized;
(c) denaturing
   - said first and its complementary strands; and
   - said second and its complementary strands; and
(d) repeating steps (a) to (c) one or more times.

It is also disclosed that the step of amplifying is performed by PCR, or PCR-like method, or RT-PCR reaction implementing the polypeptide having a DNA polymerase activity (DNA polymerase polypeptide).

"PCR" describes a method of gene amplification which involves sequenced-based hybridization of primers to specific genes within a DNA sample and subsequent amplification involving multiple rounds of annealing (hybridization), elongation and denaturation using a heat-stable DNA polymerase.

"RT-PCR" is an abbreviation for reverse transcriptase-polymerase chain reaction. Subjecting mRNA to the reverse transcriptase enzyme results in the production of cDNA which is complementary to the base sequences of the mRNA. Large amounts of selected cDNA can then be produced by means of the polymerase chain reaction which relies on the action of heat-stable DNA polymerase.

"PCR-like" will be understood to mean all methods using direct or indirect reproductions of nucleic acid sequences, or alternatively in which the labeling systems have been amplified, these techniques are of course known, in general they involve the amplification of DNA by a polymerase; when the original sample is an RNA, it is advisable to carry out a reverse transcription beforehand. There are currently a great number of methods allowing this amplification, for example the so-called NASBA "Nucleic Acid Sequence Based Amplification", TAS "Transcription based Amplification System", LCR "Ligase Chain Reaction", "Endo Run Amplification" (ERA), "Cycling Probe Reaction" (CPR), and SDA "Strand Displacement Amplification", methods well known to persons skilled in the art.

When using mRNA, the method may be carried out by converting the isolated mRNA to cDNA according to standard methods using reverse transcriptase (RT-PCR).

The present disclosure relates to a method of preparing cDNA from mRNA, comprising:
(a) contacting mRNA with an oligo(dT) primer or other complementary primer to form a hybrid, and
(b) contacting said hybrid formed in step (a) with the DNA polymerase polypeptide according to the invention and dATP, dCTP, dGTP and dTTP, whereby a cDNA-RNA hybrid is obtained.

The present disclosure is further directed to a method of preparing dsDNA (double strand DNA) from mRNA, comprising:
(a) contacting mRNA with an oligo (dT) primer or other complementary primer to form a hybrid; and
(b) contacting said hybrid formed in step (a) with the polypeptide according to the invention, dATP, dCTP, dGTP and dTTP, and an oligonucleotide or primer which is complementary to the first strand cDNA;
whereby dsDNA is obtained.

In another aspect, the present invention encompasses a method for determining the nucleotide base sequence of a DNA molecule, comprising the steps of:
(a) contacting said DNA molecule with a primer molecule able to hybridize to said DNA molecule;
(b) incubating said hybrid formed in step (a) in a vessel containing four different deoxynucleoside triphosphates, a DNA polymerase polypeptide according to the invention, and one or more DNA synthesis terminating agents which terminate DNA synthesis at a specific nucleotide base, wherein each said agent terminates DNA synthesis at a different nucleotide base; and
(c) separating the DNA products of the incubating reaction according to size, whereby at least a part of the nucleotide base sequence of said DNA can be determined.

In a preferred embodiment, said terminating agent is a dideoxynucleoside triphosphate.

A DNA synthesis terminating agent which terminates DNA synthesis at a specific nucleotide base refers to compounds, including but not limited to, dideoxynucleosides having a 2',3' dideoxy structure (e.g., ddATP, ddCTP, ddGTP and ddTTP). Any compound capable of specifically terminating a DNA sequencing reaction at a specific base may be employed as a DNA synthesis terminating agent.

In another aspect, the present invention encompasses a method for amplification of a DNA molecule comprising the steps of:
(a) incubating said DNA molecule in the presence of a polypeptide having DNA polymerase according to the invention, the terminal protein of the archaeal ampullavirus ABV and a mixture of different deoxynucleoside triphosphates.

In a preferred embodiment, the method for amplification of a DNA molecule according to the invention is **characterized in that** at one end of said DNA molecule a fragment containing the replication origin of said ABV is covalently bound.

Indeed, it is likely that ABV performs replication in a way similar to that of phi29, the sequences of the inverted terminal repeat (ITR) and the surrounding region should be involved in replication initiation. The sequences SEQ ID NO: 5 (left end) and SEQ ID NO: 6 (right end) are the sequences of both genomic termini including the ITR.

It is particulary described the sequence of the fragment containing the replication origin of said ABV comprises the sequences SEQ ID NO: 5 (left end) and SEQ ID NO: 6 (right end).

In a further aspect, the present invention is directed to a kit for sequencing a DNA molecule, comprising:
(a) a first container means comprising the polypeptide according to the invention;
(b) a second container means comprising one or more dideoxyribonucleoside triphosphates; and
(c) a third container means comprising one or more deoxyribonucleoside triphosphates.

The present invention also encompasses a kit for amplifying a DNA molecule, comprising:
(a) a first container means comprising the polypeptide according to the invention; and
(b) a second container means comprising one or more deoxyribonucleoside triphosphates.

In a more preferred embodiment, the kit for amplifying a DNA molecule according to the invention further comprises the isolated terminal protein of archaeal ampullavirus ABV corresponding to the polypeptide having the SEQ ID NO: 3 encoded by the ORF163 (SEQ ID NO: 4) of the ABV genome.

The present invention also comprises the use of a polypeptide according to the invention for implementing rolling circle amplification, multiple displacement amplification or protein-primed amplification method.

These particular methods are well known by the skilled person and are for example described in the documents:
Lizardi et al., 1998; Baner et al., 1998; Dean et al., Genome Res., 11, 1095-1099, 2001;
Larsson et al., Nature methods, 1, 227-232, 2004; for isothermal rolling-circle amplification method;
Dean et al., 2002, for multiple displacement amplification method; and
Blanco et al., 1994, for protein-primed amplification method.

It is disclosed that the method according to the invention, the kit according to the invention or the use according to the invention is **characterized in that** the DNA polymerase polypeptide according to the invention is a polypeptide having DNA polymerase activity and deficient exonuclease activity (at least less than 1 %, preferably less than 0.1 % of the activity normally associated with the wild type ABV DNA polymerase).

The exonuclease activity associated with the DNA polymerase polypeptides of the invention can not significantly interfere with the use of the polymerase in a DNA sequencing, synthesizing or amplification reaction. However, it is preferred that the level of exonuclease activity be reduced to a level which is less than 10 % or 1 %, preferably less than 0.1 % of the activity normally associated with DNA polymerases isolated from cells infected with the naturally-occuring ABV or having the sequence SEQ ID NO: 1.

Inventors also disclose an apparatus for DNA sequencing or amplification having a reactor comprising a DNA polymerase polypeptide of the present invention.

The present invention also provides methods for producing anti-DNA polymerase polypeptide of the invention comprising, exposing an animal having immunocompetent cells to an immunogen comprising a polypeptide of the invention or at least an antigenic portion (determinant) of a polypeptide of the invention under conditions such that immunocompetent cells produce antibodies directed specifically against the polypeptide of the invention, or epitopic portion thereof. In one embodiment, the method further comprises the step of harvesting the antibodies. In an alternative embodiment, the method comprises the step of fusing the immunocompetent cells with an immortal cell line under conditions such that a hybridoma is produced.

Such antibodies can be used particularly for purifying the polypeptide of the present invention in a sample where others components are present.

The following examples and the figures are given for the purpose of illustrating various embodiments of the invention and are not meant to limit the present invention in any fashion.

### Legends of the figures

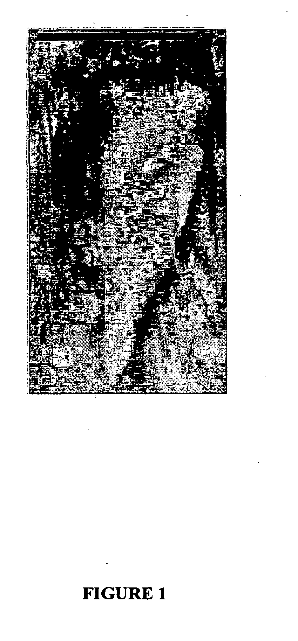

**Figure 1**. Electron micrographs of particles of ABV after negative staining with 3 % uranyl acetate. Bars, 100 nm.

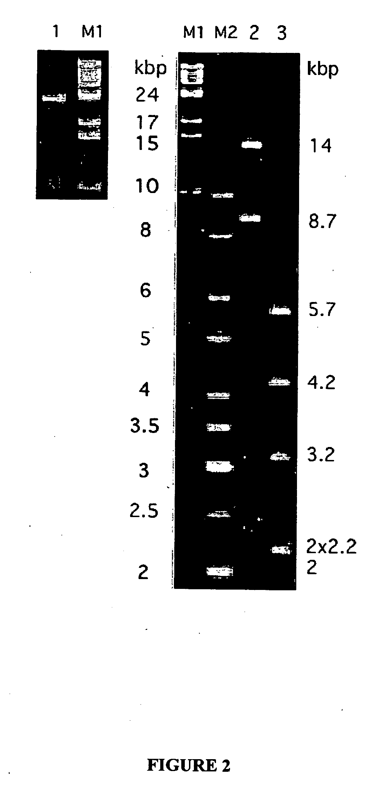

**Figure 2**. Estimation of the genome size by running intact (left panel) and restriction enzyme-digested viral DNA (right panel) in an agarose gel. Lane 1, intact viral DNA; lane 2 and 3, Age I and Afl II digested DNA respectively; M1, Lambda DNA-mono cut mix size marker from New England Biolabs (Catalog N3019S); M2, Ladder DNA size marker from Amersham.

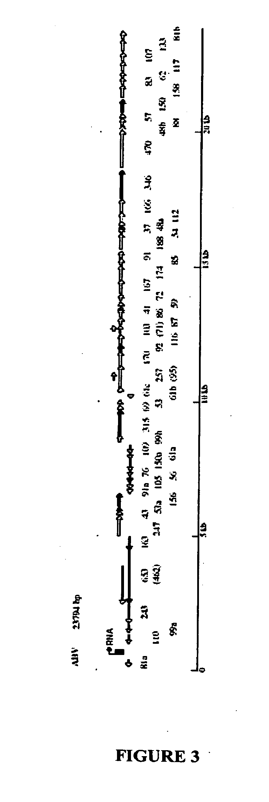

**Figure 3**. Genome map of ABV showing the location and size of the putative genes present on the two DNA strands. Most Genes are expressed on one strand as indicated by right-pointing arrows and a few on the complementary strand as shown by left-pointing arrows. Dark arrows indicate ORFs assigned functions while hypothetical genes are shown by gray arrows. Three internal ORFs are denoted by empty arrows and their sizes are in brackets. The map was drawn using MacPlasmap 2.05 and Adobe Illustrator.
**Figure 4**. Sequence alignment between ORF653 (SEQ ID NO: 1) and the Phi29 DNA polymerase (issued from SEQ ID NO: 7 (GenBank Accession number 1XI1B) which corresponds to the DNA polymerase type-B family) showing two insertions (TPR I and II) which are specific for all known protein-priming DNA polymerase sequences. The conserved motifs involved in the exonuclease activity (Exo I, II, III) and in polymerisation (Pol I, IIa, IIb, III and IV) are indicated. Numbers indicate the amino acid lengths between the sequences.

### Figures 5A and 5B:

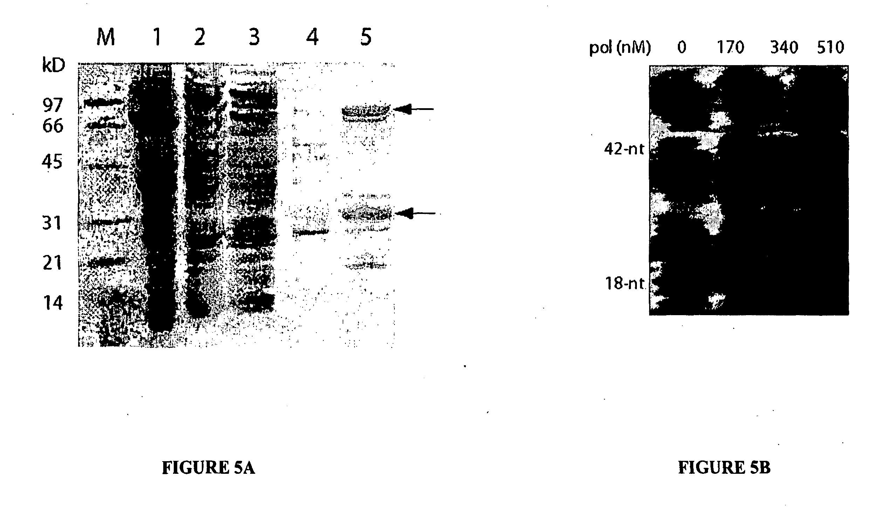

**Figure 5A**. Purification of recombinant polymerase encoded by ORF653 from E. coli. Lane 1, protein size marker; 2, total crude of the induced cells; 3, supernatant after sonicatioon and centrifugation; 4, flow through after binding of the His-tagged protein to Ni-NTA agarose resin; 5, washed-out of the resin column; 6, purified protein. The size (kD) of polypeptides in the marker is shown at the left side. Two arrows indicate the position of the intact (upper) and fragmented (lower) polymerase.
**Figure 5B****.** Polymerization assay. The concentration of polymerase in the reaction was shown on top while the position of the 18-nt primer and the elongated molecules (42-nt) is indicated at the left side.

### Figures 6A and 6B:

**Figure 6A**. Secondary structure of the putative RNA element involved in ABV packaging.
**Figure 6B****.** Secondary structure of the prohead RNA of phi29. The seven helices conserved in the bacteriophage pRNAs are labelled by A to F from 5' termini to 3' termini while original designations was made according to the lengths of the stems (Bailey et al., 1990).
   **Figure 7**. Depiction of genomic content at the left end of ABV, phi29 and adenovirus (type 5). The length of ITR is 580, 6 and 103 bp repectively. Dark box denotes the region involved in packaging, where transcription direction is shown for ABV and ∅29 by small arrows. Genes encoding polymerase (pol) and terminal protein (TP) are presented by light and dark gray arrows, respectively. Number of ORFs present between pol and the packing element is indicated in brackets.

### EXAMPLE 1: Materials and Methods

### Nucleotides, primers and enzymes

(γ-³²P)ATP, dNTPs and enzymes were obtained from Pharmacia. Oligonucleotide po1F1 (5'-CCTCCCTATTTGATAGGC-3' SEQ ID NO: 8) was 5'-labeled with (γ-³²P)ATP and T4 polynucleotide kinase and electrophoretically purifiedon 8M urea-20 % polyacrylamide gels. Labeled po1F1 was mixed with polF1c+24 (5'-AGGTAAGCATGCATCAGTTAATACGCCTATCAAATAGGGAGG-3' SEQ ID NO: 9) and the mixture was used as primer-template DNA molecule in the polymerization assay (see below).

### Purification of viruses and preparation of viral DNA

Aerobic enrichment cultures were prepared from samples taken from a water reservoir in the crater of the Solfatara volcano at Pozzuoli, Italy, at 87-93°C and pH 1.5-2, as described earlier (Häring et al., 2005a). They were grown at 75°C, pH 3. Virions were purified by centrifugation in a CsCl buoyant density gradient and disrupted with 1% (w/v) SDS for 1 hour at room temperature prior to extracting and precipitating DNA as descxribed earlier (Häring et al., 2005a).

### Sequencing of genomic DNA

Given that a total of only about 200 ng purified viral DNA was available for the project, initially, about 1 ng DNA was amplified *in vitro* to yield a few µg using the GenomiPhi amplification kit (Amersham Biotech, Amersham). A shot-gun library was then constructed from sonicated DNA fragments in the size range 1.5 to 4 kbp, cloned into the *Sma*I site of pUC18. The library produced a highly biased genome coverage.

The assembly and sequence obtained from the amplified library was confirmed by preparing a mixed shotgun library using about 50 ng original ABV DNA and 1 µg DNA extracted from *Acidianus* betalipothrixviruses (Vestergaard et al., in prep.). The library was prepared as described above except that larger sonicated DNA fragments were cloned in the range 2 to 6.5 kbp. PCR reactions were performed to verify the regions where were not covered by the second library and a few clones were also sequenced further by primer walking (Peng et al., 2001).

### Sequence analyses

BlastP search was performed against NCBI database. SMART and MotifScan were used to detect conserved domains, profiles or patterns. Coiled coils, secondary structures and transmembrane helices were detected by programs in ExPASy Proteomics Tools (http://www.expasy.org/tools/).

### Cloning and purification of the polymerase (ORF653)

ORF653 was PCR amplified from the original viral DNA using two primers containing NdeI and XhoI restriction sites, respectively (5'-TATTTTTACATATGCTACAAATCCT-3' SEQ ID NO: 10 and 5'-TATAACTCGAGTGAGAGAATACTATTTAAGTC-3' SEQ ID NO: 11). The PCR product was firstly cloned into pGEM-T vector (Promega) and the purified plasmid containing the ORF653 insert was digested with NdeI and XhoI. DNA fragment containing ORF653 with cohensive NdeI and XhoI ends was separated from pGEM-T fragment by low-melting agarose (Promega) gel electrophoresis and purified using gel extraction kit (Quiagen). The purified fragment was subsequently cloned into pET30-a vector (Novagen) digested with NdeI and XhoI and treated by calf intestinal phosphatase. The construct contains sequence encoding 6-histidine residues following the C-terminal end of the product of ORF653. Construct was sequenced to verify the sequence of the inserted ORF653 before transformation into the expression host cell Rosetta (Novagen).

A single colony of Rosetta transformant was inoculated into 5 ml LB medium containing 25 µg/ml Kanamycin and Chloramphenicol and incubated at 37°C until OD reaches 0.5. The 5 ml culture was then transformed to 250 ml LB medium containing the same antibiotics. Cells were harvested after overnight growing at 30°C in the presence of 0.1 mM IPTG and 1 % ethanol. The his-tagged protein was purified using Ni-NTA His.Bind resins according to the protocol provided by the company (Novagen) and checked by SDS-PAGE.

### Polymerization assay

The hybrid molecule polF1/polF1c+24 (described above) contains a 24-nucleotide long 5'-protuding end, and therefore can be used as primer-template for DNA polymerization. The reaction mixture contained, in 10 µl, 25 mM Tris-HCl (pH 7.6), 1 mM Dithiothreitol, 10 mM MgCl, 250 µM each of the four dNTPs, 0.1 µM of the primer-template DNA molecule and increasing concentration of recombinant polymerase. After incubation for 20 minutes at 50°C, the reaction was stopped by addition of 5 µl loading buffer (80 % formamide, 10 mM EDTA, 50 µg/ml bromophenol blue) and heating for 3 minutes at 80°C. Samples were analyzed by 8M urea-20 % PAGE and autoradiography. Polymerization was detected by as an increase in the size of the 5'-labeled primer strand (polF1).

### EXAMPLE 2: Genome sequence and organisation

Nucleic acid was isolated from ABV virions and shown to be insensitive to RNase A but digestible by type II restriction endonucleases consistent with it being ds DNA. Given the low amount of genomic DNA that was available (< 200 ng purified DNA), we adopted a two-step genome sequencing strategy.

First, about 1 ng DNA was amplified *in vitro* to yield about 2 µg DNA using the GenomiPhi amplification kit (Amersham Biotech). A shot-gun library was then constructed (see Materials and Methods) which produced a highly biased genome coverage, similar to that observed earlier for genomic DNA of the archaeal rudivirus SIRV1 which was amplified by the same procedure (Peng et al., 2004). A high level of chimeric clones were also produced. The genome was sequenced with, on average, a 20-fold coverage, and the sequences of the chimeric clones were identified by their lower frequency in the contigs and they were eliminated from the library.

In order to confirm the sequence assembly obtained from the amplified DNA library, about 50 ng of the original ABV DNA was mixed with 1 µg DNA extracted from *Acidianus* lipothrixviruses (Vestergaard et al., 2005) and a mixed shotgun library was prepared with larger cloned inserts (2 to 6.5 kbp). Sequencing and assembly of these ABV clones into those of the first library showed that the sequences from the two libraries matched exactly. Moreover, sequences of regions not covered by the second library were verified after PCR amplification of these regions, or primer walking, both performed on viral DNA and/or large insert clones. In addition, sequences of a few clones at the left terminus were extended by primer walking which yielded a final contig of about 22 kb.

To confirm the genome assembly, about 40 ng of the viral DNA was digested with the restriction enzymes *Age*I and *Afl*II. The products were fractionated by agarose gel electrophoresis together with the intact viral DNA and the bands were stained with SYBR Gold (Invitrogen). Fragment sizes were consistent with the sequence of the assembled contig and the band of the intact viral DNA indicates a genome size of about 23.8 kb (Fig. 2).

The discrepancy between the size estimate from the restriction digests and the single contig size probably reflects that terminal regions of linear viral genomes are not represented in clone libraries (Häring et al., 2004; Häring et al., 2005b). Therefore, we attempted to sequence further out from the contig ends by primer-walking on amplified DNA. This yielded about 2 kb of additional sequence beyond which sequence reads invariably terminated. The total sequence obtained was 23,794 bp, consistent with the restriction fragment digest estimate. The G+C-content was 35 %.

The genome exhibits inverted terminal repeats (ITRs) of 580 bp, smaller than those of the genomes of the rudiviruses (Peng et al., 2001) but similar to those of the archaeal betalipothrixviruses (Vestergaard et al., in prep.).

In order to test whether the genomes can circularise, PCR experiments were performed with a few different pairs of primers, annealing near each end of the genome but none of these produced an amplified product (data not shown). We infer, therefore, that the genome of ABV is linear.

### EXAMPLE 3: Gene content

The genome was annotated and start codons (88 % AUG, 6 % GUG and 6 % UUG), TATA-like promoter motifs and/or Shine-Dalgarno motifs were assigned as described earlier (Bettstetter et al., 2003, supplementary Table 1S). A map for the whole viral genome containing 59 putative ORFs ranging in size from 37 to 653 amino acids is presented in Fig. 3. Three ORFs, 653, 103 and 257, contain internal start codons which are preceded by Shine-Dalgarno motifs. The internal ORFs were thus also assigned as putative genes (Fig. 3 and Table 1). All ORFs except one are located on one strand between position 8.5 kb and the right end. Of the remainder, all except 3 (ORF247, ORF53a and ORF156) are located on the other strand between the left end and position 8.5 kb (Fig. 3). About 49 % of the ORFs shown in Fig. 3 are preceded by putative promoter sequences, and 68 % are preceded by putative Shine-Dalgarno motifs. Moreover, about 11 % of the ORFs exhibit downstream T-rich putative terminators. About 85 % of the ORFs are arranged in putative operons and about 25 % of the genes are predicted to generate transcripts that are either leaderless or carry very short leaders. The distance between ORFs is generally very short and 24 % of the ORFs overlap with upstream ORF indicating that the genome is compact. Very strikingly, the 29 ORFs located between positions 10 kb to 21 kb appear to form one single big operon (Fig. 3 and Table 1S)

Only three ORFs could be assigned unambiguous functions based on homologue searches in public sequence databases (see Materials and Methods). ORF653 showed a significant sequence similarity with family B DNA polymerases with the best matches to protein-primed polymerases. Moreover, ORF156 was identified as a thymidylate kinase and ORF315 as a putative glycosyl transferase.

All the gene annotations are summarized in Table 1. While the majority of sequenced crenarchaeal viral genomes encode at least one ribbon-helix-helix (RHH) domain protein which is the most common gene product in crenarchaeal viruses, no RHH domain was detected in the genome of ABV. However, ORF56 shows a limited similarity to tetR-type helix-turn-helix domain which is present in some prokaryotic transcription regulators involved in resistance against drugs or stress. Another three ORFs contain leucine zipper pattern which may be involved in transcription regulation (Fig. 3 and Table 1). ORF188 contains a significant EF-hand calcium-binding domain and shows limited similarity to regulatory subunit of type II protein kinase A R-subunit. Therefore, it may encode a Ca⁺⁺ dependant protein kinase. A putative apaG domain profile was detected in the sequence of ORF133 which may be involved in protein-protein interactions. The secondary structure prediction of ORF133 protein sequence revealed about 90 % extended strand and random coil. This correlates with the high content of beta-sheets in the tertiary structures of different apaG proteins. Three adjacent ORFs (112, 166 and 346) contain a few transmembrane helices and appear to be putative membrane or membrane-bound protein. Of special interest Is ORF346 which carry putative prokaryotic membrane lipoprotein lipid attachment site and EGF-like domain. The latter generally occur in the extracellular domain of membrane-bound proteins or in secreted proteins (Table 1). These properties are consistent with ORF346 constituting a viral coat protein which interacts with host membrane proteins, or a transmembrane protein which facilitates the release of viral particles from host cells. The putative transmembrane proteins encoded by the two upstream ORFs (112 and 166) might also be involved in the same process. The C-terminal sequences of both ORF346 and the downstream ORF470 show low complexity as observed in a few large ORFs in other crenarchaeal viral genomes (e.g. Häring et al., 2005; Neumann and Zillig, 1990). Function(s) of the proteins is unknown.

While three ORFs were assigned unambiguous function and a few carry putative conserved motifs or patterns (Table 1), the only gene shared between ABV and other crenarchaeal viruses is ORF315, the glycosyltransferase. Previously, comparative genomics revealed no or very few genes shared between different crenarchaeal viral genomes (Häring et al., 2004; Peng et al., 2001; Bettstetter et al., 2003). The result from this work reinforces that crenarchaeal viruses form an extremely diverse group.

**Table 1. Functions assigned to ORFs of ABV**

| ORF | Predicted function | Pfam entry or prosite document number | e-value* |
|---|---|---|---|
| 53a | Leucine zipper | Pdoc00029 | |
| 56 | HTH domain (TetR-type) | Pdoc00830 | 1.4 |
| 92a | Leucine zipper | Pdoc00029 | |
| 133 | ApaG domain profile, protein-protein interaction | Pdoc51087 | 0.94 |
| 150 | Leucine zipper | Pdoc00029 | |
| 188 | EF-Hand Ca⁺⁺ binding domain | Pdoc00018 | |
| | Regularoty subunit of type II protein kinase A R-subunit | Pfam02197 | 0.0024 |
| 346 | Lipoprotein-lipid attachment site | Pdoc00013 | |
| | EGF-like domain | Pdoc00021 | |
| 156 | Thymidylate kinase | **Pfam02223** | 6.1e-5 |
| 315 | Glycosyl transferase | Pfam00534 | 3e-10 |
| 470 | Coiled coil, protein-protein interaction | | |
| 653 | DNA polymerase | Pfam03175 | |

| | | | |
|---|---|---|---|
| *e-value is not given to hits from Prosite pattern | | | |

### EXAMPLE 4: DNA replication

With the exception of the rudiviruses, we have little insight into the replication mechanisms of archaeal viral genomes. However, ABV is exceptional in that its genome encodes a putative protein-primed DNA polymerase. These enzymes are invariably encoded in linear ds DNA genomes carrying ITRs with covalently linked terminal proteins and have been characterised in both a bacteriophage, 29, and in a eukaryal adenovirus (reviewed by Salas, 1991). The replication initiation model for these viruses involves a free terminal protein forming a heterodimer with the DNA polymerase and interacting with the replication origin via the viral DNA-bound terminal protein and specific nucleotide sequences at either end of the genome. A hydroxyl group of serine, threonine or tyrosine in the terminal protein serves as the recipient site for the first nucleotide. Moreover, many linear ds DNA plasmids and mitochondrial genomes exhibit ITRs and protein-priming DNA polymerases and carry terminal proteins which are likely to replicate in a similar way (Salas, 1991). The subfamily of protein-priming DNA polymerases belongs to the DNA-dependent DNA polymerase family B and possesses two insertions, TPR-1 and TPR-2 (Blasco et al., 2000; Dufour et al., 2000; Rodriguez et al., 2005).

A sequence alignment of ORF653 and the ∅29 DNA polymerase (Fig. 4) illustrates that ORF653 contains three exonuclease domains (Exo I, II and III) in the N-terminal region and five conserved synthetic domains (pol I, IIa, IIb, III and IV) in the C-terminal part characteristic of family B DNA polymerases (Blanco et al., 1991; Rohe et al., 1992). Moreover, the insertions TPR-1 and TPR-2 are also present in ORF653 (Fig. 4). While TPR-1 is similar in size (50 aa) to those of all the protein-priming DNA polymerases, including that encoded by human adenovirus (Dufour et al., 2000), whereas TPR-2, located between motifs pol IIa and IIb, is truncated relative to the known size range of inserts extending from 28 aa (∅29) to 118 aa (adenovirus type 2) (Bois et al., 1999). For the ∅29 DNA polymerase, TPR-1 was found to participate in the interaction with the terminal priming protein (Dufour et al., 2000) while TPR-2 was shown to be required for the high processivity and strand-displacement activity of the polymerase (Rodriguez et al., 2005). Although the function of the more conserved TPR-1 is likely to be general for all protein-priming DNA polymerases, it remains unclear whether this applies to the more variable TPR-2.

In order to confirm that ORF653 is a DNA polymerase, the gene was amplified from the viral genome by PCR and cloned into an *E*. *coli* expression vector. A reasonable amount of soluble recombinant protein was purified together with a C-terminal fragment of the protein (Fig. 5A). To test the polymerization activity, the protein was incubated with a labelled primer-template DNA at 50°C. Figure 5B clearly shows that the primer (18 nt) was elongated to the size of the template (42 nt) indicative of polymerization activity. When NTPs was used instead of dNTPs, no polymerization was detected (data not shown). This confirms that the product of ORF653 is indeed a DNA polymerase.

### EXAMPLE 5: Terminal protein

The presence of an ITR and a gene encoding a putative protein-priming DNA polymerase in the linear genome of ABV strongly suggests that each 5' terminus is covalently attached to a terminal protein. This is difficult to test experimentally, owing to the very low yields of virus particles that are produced. Therefore, we analyzed terminal protein sequences from relevant bacteriophages, linear plasmids and human adenoviruses in order to gain insights into conserved features of these proteins. While the polymerase is relatively conserved, the terminal protein shows very low conservation. For example, the terminal protein of *E. coli* bacteriophage PRD1 shows no significant sequence similarity with other known terminal proteins (Savilahti et al., 1987) and only 13/48 % identity/similarity was found between the terminal protein of ∅29 and a linear mitochondrial plasmid of white-rot fungus *Pleurotus ostreatus* (Kim et al., 2000). However, the gene location of the terminal protein is highly conserved. Thus, in bacteriophages ∅29, PRD1, GA-1 and CP-1 (Accession numbers P03681, P09009, X96987 and Z47794) and in human adenoviruses the gene is always located immediately upstream of the DNA polymerase gene whereas the size of the protein ranges between 230 aa and 266 aa for the bacteriophages and 671 aa for adenovirus type 2 (AC_000007). DNA replication was less studied for the linear plasmids, two of which were found to encode a fused N-terminal terminal protein and a C-terminal DNA polymerase (Kim et al., 2000; Takeda et al., 1996). Sequence alignment of the DNA polymerases also revealed large sequence extensions at the N-terminal part in the other linear plasmids (Bois et al., 1999), indicating that the genes of the polymerase and terminal protein may generally be fused. Moreover, transcript mapping revealed that the DNA polymerase and terminal protein genes are always cotranscribed into a single mRNA in all the studied viruses, including CP-1 (Martin et al., 1996) and ∅29 family phages (reviewed by Meijer et al., 2001). Thus, the polymerase and terminal protein are closely linked in both gene organization and function. For ABV, the gene upstream of the polymerase encodes 163 aa which is two thirds the size of the bacteriophage terminal proteins. However, sequence alignments using ClustalW (EMBL-EBI) revealed higher score between ORF163 and TP from PRD1 than between TPs of PRD1 and ∅29 (data not shown), indicating that ORF163 may encode a terminal protein.

### EXAMPLE 6: Viral DNA packaging

Earlier, it was shown that the virion structure of ABV is very complex when compared with other known crenarchaeal viruses. The bottle-shaped virion contains a "stopper" at the narrow end, and a disk, or ring, bearing 20 short filaments at the broad end (Häring et al., 2005). The main body appears to be built up of two layers encasing a complex core and the nucleoprotein filament is packed, compactly, within the main body. Thus, the DNA packaging mechanism is likely to be complex.

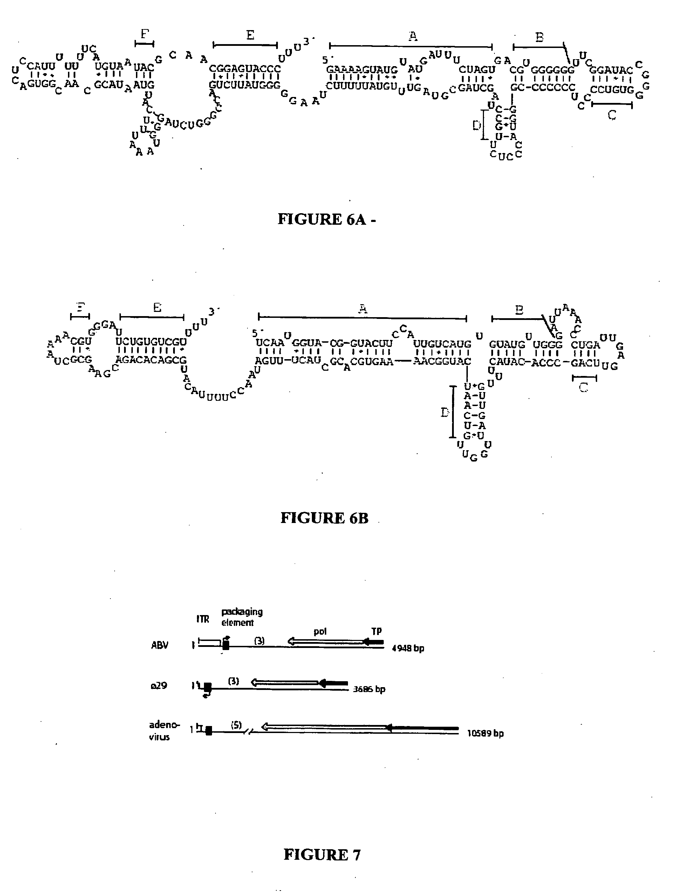

Packaging of genomic DNA has been studied for diverse bacteriophages and eukaryal viruses carrying linear genomes. The mechanisms share some common features, including the involvement of a pair of noncapsid proteins and the energy source, ATP, to translocate the long DNA molecule into a preformed procapsid (reviewed by Guo, 2005). An essential component of the ∅29 packaging machinery is a 174-nt RNA, pRNA, which participates actively in DNA translocation by binding to the procapsid and ATP and cooperating with the packaging protein (Guo, 2005). The pRNA is encoded adjacent to the ITR at one end of ∅29 genome and it exhibits a high level of secondary structure and conserved secondary structural motifs can form for all the known ∅29 related bacteriophages (reviewed by Meijer et al., 2001). Moreover, a corresponding region, adjacent to an ITR, was also found to be important for the packaging of adenoviral DNA (Grable and Hearing, 1990). Examination of the corresponding regions in the ABV genome, revealed a 600-bp region, lacking open reading frames, close to the left ITR, which was relatively G+C-rich in the centre. The predicted secondary structure for the 200 bp G+C-rich sequence shows high similarity to that of pRNA from bacteriophages ∅29 and CP-1 (Fig. 6). The seven helices labeled A to F are highly conserved in all pRNAs of bacteriophages. Differences occur only in the region to the left of helix F where extra hairpin-loops occur in the putative ABV RNA while a small loop is present in the bacteriophage pRNAs (Fig. 6). Transcription of the ABV RNA could be initiated at the promoter-like sequence, ATTTAAT, located 20 bp upstream of the element. The conserved genomic position, similar secondary structure, high G+C content and presence of a putative promoter all strongly indicate that this non-coding region encodes a RNA molecule which is probably involved in viral DNA packaging.

Another important component involved in ∅29 DNA packaging is the connector which was proposed to rotate in order to translocate the DNA into the prohead (Meijer et al., 2001). Although the general morphology of ABV is different from that of ∅29, the "stopper" resembles the connector of ∅29 which also has a bottle-neck shape and the wide end of which is also buried inside the prohead (Meijer et al., 2001). Moreover, the broad end of the stopper is connected to the nucleoprotein filament (Häring et al., 2005). Therefore, the connector may also be involved in packaging of ABV.

Currently, little is known about the packaging of archaeal virions with linear DNA. One tends to speculate that it is simpler for the crenarchaeal rudiviruses and filamentous viruses which generally have the supercoiled genomic DNA arranged in long and "linear" structures (rod or filamentous shape) containing 1 to 3 proteins. Therefore, they seem not to pack the genomic DNA into a preformed structure. For viruses as ABV and PSV, which have more compact structure and especially lengthy linear genomes, one would infer that they need a comprehensive encapsidation or packaging mechanism.

Genomic content at the left end of ABV, bacteriophage ∅29 and eukaryotic adenovirus is depicted in Figure 7 which shows high similarity between the three viruses. ABV is the first archaeal virus which is reported to contain a protein-primed DNA polymerase. The presence of the polymerase in three morphologically distinct viruses from three domains of life strongly indicates the protein-primed DNA replication mechanism is ancient, probably existed prior to the divergence of three domains of life.

### References

Altschul, S. F., T. L. Madden, A. A. Schäffer, J. Zhang, Z. Zhang, W. Miller, and D. J. Lipman. 1997. Gapped BLAST and PSI-BLAST: a new generation of protein database search programs. Nucl. Acids Res. 25:3389-3402.
Arnold, H. P., W. Zillig, U. Ziese, I. Holz, M. Crosby, T. Utterback, J. F. Weidmann, J. Kristjansson, H. P. Klenk, K. E. Nelson, and C. M. Fraser. 2000. A novel lipothrixvirus, SIFV, of the extremely thermophilic crenarchaeon Sulfolobus. Virology 267:252-266.
Barthelemy, I., M. Salas, and R. P. Mellado. 1986. In vivo transcription of bacteriophage ∅29 DNA: transcription initiation sites. J. Virol. 60:874-879.
Bettstetter, M., X. Peng, R. A. Garrett, and D. Prangishvili. 2003. AFV1, a novel virus infecting hyperthermophilic archaea of the genus Acidianus. Virology 315:68-79.
Blum, H., W. Zillig, S. Mallock, H. Domday, and D. Prangishvili. 2001. The genome of the archaeal virus SIRV1 has features in common with genomes of eukaryal viruses. Virology 281:6-9.
Blanco L, Lazaro JM, de Vega M, Bonnin A, Salas M. Terminal protein-primed DNA amplification. Proc Natl Acad Sci U S A. 1994 Dec 6;91(25):12198-202.
Bois F, Barroso G, Gonzalez P, Labarere J. 1999. Molecular cloning, sequence and expression of Aa-polB, a mitochondrial gene encoding a family B DNA polymerase from the edible basidiomycete Agrocybe aegerita. Mol. Gen. Genet. 261:508-513.
Bravo, A., B. Illana, and M. Salas. 2000. Compartmentalization of phage ∅29 DNA replication: interaction between the primer terminal protein and the membrane-associated protein p1. EMBO J. 19:5575-5584.
Brugger, K., P. Redder, and M. Skovgaard. 2003. MUTAGEN: Multi User Tool for Annotating GENomes. Bioinformatics 19:2480-2481.
Dufour, E, J. Mendez, J. M. Lazaro, M. de Vega, L. Blanco and M. Salas. 2000. An aspartic acid residue in TPR-1, a specific region of protein-priming DNA polymerases, is required for the functional interaction with primer terminal protein. J. Mol. Biol. 304:289-300.
Guo P. 2005. Bacterial virus phi29 DNA-packaging motor and its potential applications in gene therapy and nanotechnology. Methods Mol Biol. 300:285-324.
Grable M, Hearing P. 1990. Adenovirus type 5 packaging domain is composed of a repeated element that is functionally redundant. J. Virol. 64:2047-56.
Hatfield, L., and P. Hearing. 1993. The NFIII/OCT-1 binding site stimulates adenovirus DNA replication in vivo and is functionally redundant with adjacent sequences. J. Virol. 67:3931-3939.
Häring, M., X. Peng, K. Brugger, R. Rachel, K.O. Stetter, R.A. Garrett, and D. Prangishvili. 2004. Morphology and genome organization of the virus PSV of the hyperthermophilic archaeal genera Pyrobaculum and Thermoproteus: a novel virus family, the Globuloviridae. Virology 323:233-242.
Häring, M., R. Rachel, X. Peng, R.A. Garrett, and D. Prangishvili 2005a. Diverse viruses in hot springs of Pozzuoli, Italy, including a unique bottle-shaped archaeal virus ABV from a new family, the Ampullaviridae. J. Virol. 79:9904-9911.
Häring, M., Vestergaard, G., Brugger, K., Rachel, R., Garrett, R. A. and Prangishvili, D. 2005b. Structure and genome organisation of AFV2, a novel filamentous archaeal virus with unusual terminal structures. J. Bacteriol. 187:3855-3858.
Häring, M. et al., 2005c. Nature in press.
Janekovic, D., S. Wunderl, I. Holz, W. Zillig, A. Gierl, and H. Neumann. 1983. TTV1, TTV2 and TTV3, a family of viruses of the extremely thermophilic, anaerobic sulfur reducing archaebacterium Thermoproteus tenax. Mol. Gen. Genet. 192:39-45.
Kim, E. K., J. H. Jeong., H. S. Youn, Y. B. Koo and J. H. Roe. 2000. The terminal protein of a linear mitochondrial plasmid is encoded in the N-terminus of the DNA polymerase gene in white-rot fungus Pleurotus ostreatus. Curr. Genet. 38:283-290.
Martin, A. C., R. Lopez and P. Garcia. 1996. Analysis of the complete nucleotide sequence and functional organization of the genome of Streptococcus pneumoniae bacteriophage Cp-1. J Virol. 70:3678-3687.
Meijer, W. J., J. A. Horcajadas and M. Salas. 2001. Phi29 family of phages. Microbiol. Mol. Biol. Rev. 65:261-287.
Peng, X., H. Blum, Q. She, S. Mallok, K. Brügger, R.A. Garrett, W. Zillig, and D. Prangishvili. 2001. Sequences and replication of genomes of the archaeal rudiviruses SIRV1 and SIRV2: Relationships to the archaeal lipothrixvirus SIFV and some eukaryal viruses. Virology 291: 226-234.
Peng, X., A. Kessler, H. Phan, R. A. Garrett, and D. Prangishvili. 2004. Multiple variants of the archaeal DNA rudivirus SIRV1 in a single host and a novel mechanism of genome variation. Mol. Microbiol. 54:366-375.
Picardeau, M., J. R. Lobry, and B. J. Hinnebusch. 1999. Physical mapping of an origin of bidirectional replication at the centre of the Borrelia burgdorferi linear chromosome. Mol. Microbiol. 32:437-445.
Prangishvili, D., K. M. Stedman, and W. Zillig. 2001. Viruses of the extremely thermophilic archaeon Sulfolobus. Trends Microbiol. 9:39-42.
Prangishvili, D. and R. A. Garrett. 2004. Exceptionally diverse morphotypes and genomes of crenarchaeal hyperthermophilic viruses. Biochem. Soc. Trans. 32:204-208.
Rachel, R., M. Bettstetter, B.P. Hedlund, M. Häring, A. Kessler, K.O. Stetter, and D. Prangishvili. 2002. Remarkable morphological diversity of viruses and virus-like particles in terrestrial hot environments. Arch. Virol. 147:2419-2429.
Rodriguez, I., J. M. Lazaro, L. Blanco, S. Kamtekar, A. J. Berman, J. Wang, T. A. Steitz, M. Salas, and M. de Vega. 2005. A specific subdomain in phi29 DNA polymerase confers both processivity and strand-displacement capacity. Proc. Natl. Acad. Sci. USA. 102:6407-12.
Salas, M. 1991. Protein-priming of DNA replication. Annu. Rev. Biochem. 60:39-71.
Savilahti H. and D.H. Bamford. 1987. The complete nucleotide sequence of the left very early region of Escherichia coli bacteriophage PRD1 coding for the terminal protein and the DNA polymerase. Gene 57:121-130.
Takeda M., H. Hiraishi, T. Takesako, S. Tanase and N. Gunge. 1996. The terminal protein of the linear DNA plasmid pGKL2 shares an N-terminal domain of the plasmid-encoded DNA polymerase. Yeast 12:241-246.
Torarinsson, E., H.-P. Klenk, and R. A. Garrett. 2005. Divergent transcriptional and translational signals in Archaea. Environ. Microbiol. 7:47-54.
Vestergaard, G., M. Haering, X. Peng, R. Rachel, R. A. Garrett, and D. Prangishvili. 2005. ARV1, a rudivirus infecting the hyperthermophilic archaeal genus Acidianus. Virology, 336:83-92.
Zillig, W., D. Prangishvili, C. Schleper, M. Elferink, I. Holz, S. Albers, D. Janekovic, and D. Götz. 1996. Viruses, plasmids and other genetic elements of thermophilic and hyperthermophilic Archaea. FEMS Microbiol. Rev. 18:225-236.

## Claims

1. An isolated DNA polymerase selected from the group of polypeptides consisting of:
a) the polypeptide having the amino acid sequence of SEQ ID NO: 1;
b) a fragment of a) having a DNA polymerase activity;
c) a polypeptide comprising at least the SEQ ID NO: 1 fragments allowing the DNA polymerase activity of said DNA polymerase of a);
d) a polypeptide having the amino acid sequence of SEQ ID NO: 1 wherein the following exonuclease sites as identified in Figure 4:
Exo I: ---I---DLET---,
Exo II: -Y-HNL-FD---FIL, and/or
Exo III: KE---YL--D---L,
have been mutated or deleted in order that the resulting DNA polymerase polypeptide has deficient or no detectable exonuclease activity compared to the polypeptide having the amino acid sequence of SEQ ID NO: 1;
e) a polypeptide having sequence which is at least 80 % identity after optimum alignment with the sequence SEQ ID NO: 1, said polypeptide having a DNA polymerase activity.

2. The DNA polymerase of claim 1, which is isolated from the Archaeal Ampullavirus ABV.

3. The DNA polymerase of claim 1 or 2, which comprises at least the following fragments of SEQ ID NO: 1 as identified in Figure 4:
Pol I: YDVNSLYP-AM;
Pol IIa: --K---NS-YG;
Pol IIb: T---GR;
Pol III: Y-DTDS; and
Pol IV: K-Y.

4. A nucleic acid encoding a DNA polymerase polypeptide according to one of claims 1 to 3.

5. A vector comprising the nucleic acid of claim 4.

6. The vector of claim 5, wherein said nucleic acid is operably linked to a promoter.

7. The vector of claim 5, which has been deposited at the C.N.C.M. (Collection Nationale de Cultures de Microorganismes, Institut Pasteur, Paris, France) the 28 April 2006 under the number I-3601.

8. A host cell comprising the vector of claims 5 to 7.

9. The host cell of claim 8, which has been deposited at the C.N.C.M. (Collection Nationale de Cultures de Microorganismes, Institut Pasteur, Paris, France) the 28 April 2006 under the number I-3601.

10. A method of producing a DNA polymerase, said method comprising:
(a) culturing the host cell of claim 8 or 9 in conditions suitable for the expression of said nucleic acid; and
(b) isolating said DNA polymerase from said host cell.

11. The method of claim 10, wherein said host cell is a prokaryotic or an eukaryotic cell.

12. A method of synthesizing a double-stranded DNA molecule comprising:
(a) hybridizing a primer to a first DNA molecule; and
(b) incubating said DNA molecule of step (a) in the presence of one or more deoxyribonucleoside triphosphates or analogs thereof and the polypeptide of claims 1 to 3, under conditions sufficient to synthesize a second DNA molecule complementary to all or a portion of said first DNA molecule.

13. A method of synthesizing a single-stranded DNA molecule comprising:
(a) the synthesis of a double-stranded DNA molecule by a method according to claim 12; and
(b) denaturing the double-stranded DNA molecule obtained in step (a); and
(c) recovering the single-stranded DNA molecule obtained in step (b).

14. A method for production of DNA molecules of greater than 10 kilobases in length comprising the methods of claims 12 or 13, wherein the first DNA molecule:
which serve as a template in step (a) is greater than 10 kilobases.

15. The method of claims 12 to 14, wherein said deoxyribonucleoside triphosphates are selected from the group consisting of dATP, dCTP, dGTP and dTTP.

16. A method for amplifying a double stranded DNA molecule, comprising:
(a) providing a first and second primer, wherein said first primer is complementary to a sequence at or near the 3'-termini of the first strand of said DNA molecule and said second primer is complementary to a sequence at or near the 3'-termini of the second strand of said DNA molecule;
(b) hybridizing said first primer to said first strand and said second primer to said second strand in the presence of the polypeptide of claims 1 to 3, under conditions such that a nucleic acid complementary to said first strand and a nucleic acid complementary to said second strand are synthesized;
(c) denaturing
- said first and its complementary strands; and
- said second and its complementary strands; and
(d) repeating steps (a) to (c) one or more times.

17. A method for determining the nucleotide base sequence of a DNA molecule, comprising the steps of:
(a) contacting said DNA molecule with a primer molecule able to hybridize to said DNA molecule;
(b) incubating said hybrid formed in step (a) in a vessel containing four different deoxynucleoside triphosphates, a DNA polymerase polypeptide of claims 1 to 3, and one or more DNA synthesis terminating agents which terminate DNA synthesis at a specific nucleotide base, wherein each said agent terminates DNA synthesis at a different nucleotide base; and
(c) separating the DNA products of the incubating reaction according to size, whereby at least a part of the nucleotide base sequence of said DNA can be determined.

18. The method of claim 17, wherein said terminating agent is a dideoxynucleoside triphosphate.

19. A method for amplification of a DNA molecule comprising the steps of:
(a) incubating said DNA molecule in the presence of a polypeptide having DNA polymerase of claims 1 to 3, the terminal protein of the archaeal ampullavirus ABV and a mixture of different deoxynucleoside triphosphates.

20. A method for amplification of a DNA molecule according to claim 19, wherein at one end of said DNA molecule a fragment containing the replication origin of said ABV is covalently bound.

21. The method of claims 10 to 20, wherein the polypeptide of claims 1 to 3 is a polypeptide as defined in claim 1d) having deficient exonuclease activity and a DNA polymerase activity.

22. A kit for sequencing a DNA molecule, comprising:
(a) a first container means comprising the polypeptide of claims 1 to 3;
(b) a second container means comprising one or more dideoxyribonucleoside triphosphates; and
(c) a third container means comprising one or more deoxyribonucleoside triphosphates.

23. A kit for amplifying a DNA molecule, comprising:
(a) a first container means comprising the polypeptide of claims 1 to 3; and
(b) a second container means comprising one or more deoxyribonucleoside triphosphates.

24. A kit of claim 23, further comprising an isolated or recombinant terminal protein of archaeal ampullavirus ABV having the sequence SEQ ID NO: 3.

25. The kit of claims 22 to 24, wherein the polypeptide of claims 1 to 3 is a polypeptide as defined in claim 1d) having deficient exonuclease activity and a DNA polymerase activity.

26. Use of a polypeptide of claims 1 to 3 for rolling circle amplification, multiple displacement amplification or protein-primed amplification.

27. The use of claim 26, wherein the polypeptide of claims 1 to 3 is a polypeptide as defined in claim 1d) having deficient exonuclease activity and a DNA polymerase activity.

## Patentansprüche

1. Isolierte DNA-Polymerase, ausgewählt aus der Gruppe von Polypeptiden bestehend aus:
a) dem Polypeptid mit der Aminosäuresequenz SEQ ID NR.: 1;
b) einem Fragment von a) mit einer DNA-Polymerase-Aktivität;
c) einem Polypeptid, das wenigstens die SEQ ID NR.: 1 Fragmente umfasst, welche die DNA-Polymerase-Aktivität der DNA-Polymerase von a) ermöglichen;
d) einem Polypeptid mit der Aminosäuresequenz SEQ ID NR.: 1, wobei die folgenden Exonuclease-Stellen, wie in Figur 4 definiert,
Exo I: ---I---DLET---,
Exo II: -Y-HNL-FD---FIL, und/oder
Exo III: KE---YL--D---L,
mutiert oder deletiert wurden, damit das resultierende DNA-Polymerase-Polypeptid eine defiziente oder nicht nachweisbare Exonuclase-Aktivität im Vergleich zu dem Polypeptid mit der Aminosäuresequenz SEQ ID NR.: 1 aufweist;
e) einem Polypeptid mit einer Sequenz, die nach einer optimalen Angleichung einen Grad der Ähnlichkeit mit der Sequenz SEQ ID NR.: 1 von mindestens 80 % aufweist, wobei das Polypeptid eine DNA-Polymerase-Aktivität aufweist.

2. DNA-Polymerase nach Anspruch 1, die vom archaeellen Ampullavirus ABV isoliert ist.

3. DNA-Polymerase nach Anspruch 1 oder 2, die wenigstens die folgenden Fragmente von SEQ ID NR.: 1, wie in Figur 4 definiert, umfasst:
Pol I: YDVNSLYP-AM;
Pol IIa: --K---NS-YG;
Pol IIb: T---GR;
Pol III: Y-DTDS; und
Pol IV: K-Y.

4. Nucleinsäure, die ein DNA-Polymerase-Polypeptid nach einem der Ansprüche 1 bis 3 codiert.

5. Vektor, der die Nucleinsäure nach Anspruch 4 umfasst.

6. Vektor nach Anspruch 5, wobei die Nucleinsäure funktionsfähig mit einem Promoter gekoppelt ist.

7. Vektor nach Anspruch 5, der am 28. April 2006 unter der Nummer 1-3601 im C.N.C.M. (Collection Nationale de Cultures de Microorganismes, Institut Pasteur, Paris, Frankreich) hinterlegt wurde.

8. Wirtszelle, die den Vektor nach Anspruch 5 bis 7 umfasst.

9. Wirtszelle nach Anspruch 8, die am 28. April 2006 unter der Nummer 1-3601 im C.N.C.M. (Collection Nationale de Cultures de Microorganismes, Institut Pasteur, Paris, Frankreich) hinterlegt wurde.

10. Verfahren zur Erzeugung einer DNA-Polymerase, wobei das Verfahren umfasst:
(a) Kultivieren der Wirtszelle nach Anspruch 8 oder 9 unter Bedingungen, die für die Expression der Nucleinsäure geeignet sind; und
(b) Isolieren der DNA-Polymerase von der Wirtszelle.

11. Verfahren nach Anspruch 10, wobei es sich bei der Wirtszelle um eine prokaryotische oder eine eukaryotische Zelle handelt.

12. Verfahren zur Synthese eines doppelsträngigen DNA-Moleküls, umfassend:
(a) Hybridisieren eines Primers an ein erstes DNA-Molekül; und
(b) Inkubieren des DNA-Moleküls von Schritt (a) in Gegenwart von einem oder mehreren Desoxyribonucleosidtriphosphaten oder Analogen davon und des Polypeptids nach Anspruch 1 bis 3 unter Bedingungen, die ausreichen, um ein zweites DNA-Molekül komplementär zur Gesamtheit oder einem Teil des ersten DNA-Moleküls zu synthetisieren.

13. Verfahren zur Synthese eines einsträngigen DNA-Moleküls, umfassend:
(a) die Synthese eines doppelsträngigen DNA-Moleküls durch ein Verfahren nach Anspruch 12; und
(b) Denaturieren des in Schritt a) erhaltenen doppelsträngigen DNA-Moleküls; und
(c) Gewinnen des in Schritt b) erhaltenen einsträngigen DNA-Moleküls.

14. Verfahren zur Erzeugung von DNA-Molekülen mit einer Länge von mehr als 10 Kilobasen, umfassend die Verfahren nach Anspruch 12 oder 13, wobei das erste DNA-Molekül, das in Schritt a) als eine Matrize dient, länger als 10 Kilobasen ist.

15. Verfahren nach Anspruch 12 bis 14, wobei die Desoxyribonucleotidtriphosphate aus der Gruppe bestehend aus dATP, dCTP, dGTP und dTTP ausgewählt sind.

16. Verfahren zur Amplifikation eines doppelsträngigen DNA-Moleküls, umfassend:
(a) Bereitstellen eines ersten und zweiten Primers, wobei der erste Primer komplementär zu einer Sequenz an oder in der Nähe der 3'-Termini des ersten Stranges des DNA-Moleküls ist, und der zweite Primer komplementär zu einer Sequenz an oder in der Nähe der 3'-Termini des zweiten Stranges des DNA-Moleküls ist;
(b) Hybridisieren des ersten Primers an den ersten Strang und des zweiten Primers an den zweiten Strang in Gegenwart des Polypeptids nach Anspruch 1 bis 3 unter derartigen Bedingungen, dass eine Nucleinsäure komplementär zum ersten Strang und eine Nucleinsäure komplementär zum zweiten Strang synthetisiert werden;
(c) Denaturieren
- des ersten und seiner komplementären Stränge; und
- des zweiten und seiner komplementären Stränge; und
(d) Wiederholen der Schritte (a) bis (c) einmal oder mehrere Male.

17. Verfahren zur Bestimmung der Nucleotidbasensequenz eines DNA-Moleküls, umfassend die folgenden Schritte:
(a) Kontaktieren des DNA-Moleküls mit einem Primermolekül, das zum Hybridisieren an das DNA-Molekül fähig ist;
(b) Inkubieren des in Schritt (a) gebildeten Hybrids in einem Gefäß, das vier verschiedene Desoxynucleosidtriphosphate, ein DNA-Polymerase-Peptid nach Anspruch 1 bis 3 und ein oder mehrere DNA-Synthese-terminierende Agenzien enthält, welche die DNA-Synthese an einer spezifischen Nucleotidbase terminieren, wobei jedes der Agenzien die DNA-Synthese an einer verschiedenen Nucleotidbase terminiert; und
(c) Trennen der DNA-Produkte der Inkubationsreaktion nach der Größe, wodurch wenigstens ein Teil der Nucleotidbasensequenz der DNA bestimmt werden kann.

18. Verfahren nach Anspruch 17, wobei es sich bei dem terminierenden Agens um ein Didesoxynucleosidtriphosphat handelt.

19. Verfahren zur Amplifikation eines DNA-Moleküls, umfassend den folgenden Schritt:
(a) Inkubieren des DNA-Moleküls in Gegenwart eines Polypeptids mit einer DNA-Polymerase nach Anspruch 1 bis 3, des terminalen Proteins des archaeellen Ampullavirus ABV und eines Gemisches verschiedener Desoxynucleosidtriphosphate.

20. Verfahren zur Amplifikation eines DNA-Moleküls nach Anspruch 19, wobei an ein Ende des DNA-Moleküls kovalent ein Fragment gebunden ist, das den Replikationsstartpunkt des ABV enthält.

21. Verfahren nach Anspruch 10 bis 20, wobei es sich bei dem Polypeptid nach Anspruch 1 bis 3 um ein Polypetid wie in Anspruch 1d) definiert mit einer defizienten Exonuclease-Aktivität und einer DNA-Polymerase-Aktivität handelt.

22. Kit zur Sequenzierung eines DNA-Moleküls, umfassend:
(a) ein erstes Behältermittel, welches das Polypeptid Anspruch 1 bis 3 umfasst;
(b) ein zweites Behältermittel, das ein oder mehrere Didesoxyribonucleosidtriphosphate umfasst; und
(c) ein drittes Behältermittel, das ein oder mehrere Desoxyribonucleosidtriphosphate umfasst.

23. Kit zur Amplifikation eines DNA-Moleküls, umfassend:
(a) ein erstes Behältermittel, welches das Polypeptid Anspruch 1 bis 3 umfasst;
(b) ein drittes Behältermittel, das ein oder mehrere Desoxyribonucleosidtriphosphate umfasst.

24. Kit nach Anspruch 23, ferner umfassend ein isoliertes oder rekombinantes terminales Protein des archaeellen Ampullavirus ABV mit der Sequenz SEQ ID NR.: 3.

25. Kit nach Anspruch 22 bis 24, wobei es sich bei dem Polypeptid nach Anspruch 1 bis 3 um ein Polypetid wie in Anspruch 1d) definiert mit einer defizienten Exonuclease-Aktivität und einer DNA-Polymerase-Aktivität handelt.

26. Verwendung eines Polypeptids nach Anspruch 1 bis 3 zur Amplifikation am rollenden Kreis oder multiplen Verdrängungsamplifikation oder proteinprimerunterstützten Amplifikation.

27. Kit nach Anspruch 26, wobei es sich bei dem Polypeptid nach Anspruch 1 bis 3 um ein Polypetid wie in Anspruch 1d) definiert mit einer defizienten Exonuclease-Aktivität und einer DNA-Polymerase-Aktivität handelt.

## Revendications

1. ADN polymérase isolée choisie dans le groupe de polypeptides constitué par :
a) le polypeptide ayant la séquence d'acides aminés de SEQ ID No.: 1 ;
b) un fragment de a) ayant une activité ADN polymérase ;
c) un polypeptide comprenant au moins les fragments de SEQ ID No.: 1 permettant l'activité ADN polymérase de ladite ADN polymérase de a) ;
d) un polypeptide ayant la séquence d'acides aminés de SEQ ID No.: 1, dans lequel les sites d'exonucléases suivants, identifiés sur la figure 4 :
Exo I : ---I---DLET---,
Exo II : -Y-HNL-FD---FIL, et/ou
Exo III : KE---YL--D---L,
ont été mutés ou délétés pour que le polypeptide d'ADN polymérase obtenu ait un déficit d'activité exonucléase, voire pas d'activité exonucléase détectable, par rapport au polypeptide ayant la séquence d'acides aminés de SEQ ID No.: 1 ;
e) un polypeptide ayant une séquence qui présente une identité d'au moins 80 %, après alignement optimal, avec la séquence SEQ ID No.:1, ledit polypeptide ayant une activité ADN polymérase.

2. ADN polymérase selon la revendication 1, qui est isolée à partir de l'ampullavirus d'Archaea ABV.

3. ADN polymérase selon les revendications 1 ou 2, qui comprend au moins les fragments suivants de SEQ ID No.: 1 comme identifié sur la figure 4 :
Pol I : YDVNSSLYP-AM ;
Pol IIa : --K---NS-YG ;
Pol IIb : T---GR ;
Pol III : Y-DTDS ; et
Pol IV : K-Y.

4. Acide nucléique codant pour un polypeptide d'ADN polymérase selon l'une des revendications 1 à 3.

5. Vecteur comprenant l'acide nucléique selon la revendication 4.

6. Vecteur selon la revendication 5, dans lequel ledit acide nucléique est lié de manière opérationnelle à un promoteur.

7. Vecteur selon la revendication 5, qui a été déposé auprès de la C.N.C.M. (Collection Nationale de Cultures de Microorganismes, Institut Pasteur, Paris, France) le 28 Avril 2006 sous le numéro 1-3601.

8. Cellule hôte comprenant le vecteur selon les revendications 5 à 7.

9. Cellule hôte selon la revendication 8, qui a été déposée auprès de la C.N.C.M. (Collection Nationale de Cultures de Microorganismes, Institut Pasteur, Paris, France) le 28 Avril 2006 sous le numéro 1-3601.

10. Procédé de production d'une ADN polymérase, ledit procédé comprenant :
(a) la culture de la cellule hôte selon les revendications 8 ou 9 dans des conditions convenables pour l'expression dudit acide nucléique ; et
(b) l'isolement de ladite ADN polymérase à partir de ladite cellule hôte.

11. Procédé selon la revendication 10, dans lequel ladite cellule hôte est une cellule procaryote ou eucaryote.

12. Procédé de synthèse d'une molécule d'ADN double brin comprenant :
(a) l'hybridation d'une amorce à une première molécule d'ADN ; et
(b) l'incubation de ladite molécule d'ADN de l'étape (a) en présence d'un ou de plusieurs désoxyribonucléosides triphosphates ou analogues de ceux-ci et du polypeptide selon les revendications 1 à 3, dans des conditions suffisantes pour synthétiser une seconde molécule d'ADN complémentaire de tout ou partie de ladite première molécule d'ADN.

13. Procédé de synthèse d'une molécule d'ADN simple brin comprenant :
(a) la synthèse d'une molécule d'ADN double brin par un procédé selon la revendication 12; et
(b) la dénaturation de la molécule d'ADN double brin obtenue à l'étape (a) ; et
(c) la récupération de la molécule d'ADN simple brin obtenue à l'étape (b).

14. Procédé de production de molécules d'ADN de plus de 10 kilobases de long comprenant les procédés selon les revendications 12 ou 13, dans lequel la première molécule d'ADN qui sert de matrice dans l'étape (a) est supérieure à 10 kilobases.

15. Procédé selon les revendications 12 à 14, dans lequel lesdits désoxyribonucléosides triphosphates sont choisis dans le groupe constitué par les dATP, dCTP, dGTP et dTTP.

16. Procédé d'amplification d'une molécule d'ADN double brin, comprenant :
(a) l'utilisation d'une première et d'une seconde amorces, ladite première amorce étant complémentaire d'une séquence au niveau, ou à proximité des terminaisons 3' du premier brin de ladite molécule d'ADN et ladite seconde amorce étant complémentaire d'une séquence au niveau, ou à proximité des terminaisons 3' du second brin de ladite molécule d'ADN ;
(b) l'hybridation de ladite première amorce audit premier brin et de ladite seconde amorce audit second brin en présence du polypeptide selon les revendications 1 à 3, dans des conditions telles qu'un acide nucléique complémentaire dudit premier brin et un acide nucléique complémentaire dudit second brin sont synthétisés ;
(c) la dénaturation
- dudit premier brin et de son brin complémentaire ; et
- dudit second brin et de son brin complémentaire ; et
(d) la répétition des étapes (a) à (c) une ou plusieurs fois.

17. Procédé permettant de déterminer la séquence de bases nucléotidiques d'une molécule d'ADN, comprenant les étapes suivantes :
(a) la mise en contact de ladite molécule d'ADN avec une molécule d'amorce capable de s'hybrider à ladite molécule d'ADN ;
(b) l'incubation dudit hybride formé à l'étape (a) dans un réacteur contenant quatre désoxynucléosides triphosphates différents, un polypeptide d'ADN polymérase selon les revendications 1 à 3, et un ou plusieurs agents de terminaison de synthèse de l'ADN qui terminent la synthèse de l'ADN à une base nucléotidique spécifique, chacun desdits agents terminant la synthèse de l'ADN à une base nucléotidique différente ; et
(c) la séparation des produits d'ADN de la réaction d'incubation en fonction de la taille, de façon qu'au moins une partie de la séquence de bases nucléotidiques dudit ADN puisse être déterminée.

18. Procédé selon la revendication 17, dans lequel ledit agent de terminaison est un didésoxy-nucléoside triphosphate.

19. Procédé d'amplification d'une molécule d'ADN comprenant les étapes suivantes :
(a) l'incubation de ladite molécule d'ADN en présence d'un polypeptide contenant l'ADN polymérase selon les revendications 1 à 3, de la protéine terminale de l'ampullavirus d'Archaea ABV et d'un mélange de désoxynucléosides triphosphates différents.

20. Procédé d'amplification d'une molécule d'ADN selon la revendication 19, dans lequel à une extrémité de ladite molécule d'ADN un fragment contenant l'origine de réplication dudit ABV est lié par covalence.

21. Procédé selon les revendications 10 à 20, dans lequel le polypeptide selon les revendications 1 à 3 est un polypeptide tel que défini dans la revendication 1d) ayant un déficit d'activité exonucléase et une activité ADN polymérase.

22. Kit pour le séquençage d'une molécule d'ADN, comprenant :
(a) un premier moyen de récipient comprenant le polypeptide selon les revendications 1 à 3 ;
(b) un deuxième moyen de récipient comprenant un ou plusieurs didésoxyribonucléosides triphosphates ; et
(c) un troisième moyen de récipient comprenant un ou plusieurs désoxyribonucléosides triphosphates.

23. Kit pour l'amplification d'une molécule d'ADN, comprenant :
(a) un premier moyen de récipient comprenant le polypeptide selon les revendications 1 à 3 ; et
(b) un second moyen de récipient comprenant un ou plusieurs désoxyribonucléosides triphosphates.

24. Kit selon la revendication 23 comprenant, en outre, une protéine terminale isolée ou recombinante d'ampullavirus d'Archaea ABV ayant la séquence SEQ ID No.: 3.

25. Kit selon les revendications 22 à 24, dans lequel le polypeptide selon les revendications 1 à 3 est un polypeptide tel que défini dans la revendication 1d) ayant un déficit d'activité exonucléase et une activité ADN polymérase.

26. Utilisation d'un polypeptide selon les revendications 1 à 3 pour l'amplification en cercle roulant, l'amplification par déplacements multiples ou l'amplification avec une amorce protéique.

27. Utilisation selon la revendication 26, dans laquelle le polypeptide selon les revendications 1 à 3 est un polypeptide tel que défini dans la revendication 1d) ayant un déficit d'activité exonucléase et une activité ADN polymérase.
